# FASCICULE DE BREVET EUROPEEN

(11) **EP 1 078 078 B1**
(45) Date de publication et mention de la délivrance du brevet: **13.12.2006**
(21) Numéro de dépôt: 99918065.6
(22) Date de dépôt: 12.05.1999
(51) Int. Cl.: C12N 15/62, C07K 14/705, C07K 16/00, A61K 38/00

(54) **COMPLEXE FORME D'UN PEPTIDE ET D'UN PRODUIT DU COMPLEXE MAJEUR D'HISTOCOMPTABILITE A LA SURFACE DE PHAGES**
KOMPLEX AUS PEPTID UND MHC-PEPTID AUF OBERFLÄCHE VON PHAGEN
COMPLEX FORMED BY A PEPTIDE AND A MAJOR HISTOCOMPATIBILITY COMPLEX AT THE SURFACE OF PHAGES

(30) Priorité: 14.05.1998 FR 9806213
(43) Date de publication de la demande: 28.02.2001
(73) Titulaire: CENTRE NATIONAL DE LA RECHERCHE SCIENTIFIQUE, 75794 Paris Cedex (FR)
(72) Inventeur: GOROCHOV, Guy, F-75013 Paris (FR); PIQUERAS, Bernard, F-75012 Paris (FR); LE DOUSSAL, Jean-Marc, F-75008 Paris Cedex (FR); DEBRE, Patrice, F-75006 Paris (FR)
(74) Mandataire: Breese, Pierre
(86) Numéro de dépôt international: PCT/FR1999/001147
(87) Numéro de publication internationale: WO 1999/058693

(56) Documents cités:
- WO-A-91/12332
- WO-A-96/04314
- WO-A-96/40944
- WO-A-97/02342
- WO-A-97/08328
- WO-A-97/15669
- WO-A-98/03552
- WO-A-98/06749
- DE-A- 4 224 542
- US-A- 5 734 023

## Description

La présente invention concerne phage présentant à sa surface un complexe formé d'un peptide (P) et d'un produit du Complexe Majeur d'Histocompatibilité (CMH).

Plus particulièrement, la présente invention a pour objet un procédé de préparation d'un tel phage.

La présente invention se rapporte encore aux phages ainsi obtenus et à leur utilisation notamment pour le criblage de peptides reconnus par les cellules T.

Les cellules T jouent un rôle essentiel dans l'immunité, en particulier dans l'auto-immunité, la surveillance anti-tumorale, le rejet de greffe et dans la protection contre les infections par des virus et d'autres pathogènes intracellulaires. Grâce à leur récepteur à l'antigène (1) hautement variable, les cellules T reconnaissent les peptides antigéniques (P) associés aux produits du complexe majeur d'histocompatibilité (2), et présentés à la surface des cellules. Les peptides antigéniques comportent en général 8 à 12 acides aminés (3) issus de l'apprêtage intracellulaire des protéines. Il y a deux types majeurs de CMH : classe I et classe II. Les CMH de classe I (CMH-I) sont composés d'une chaîne α de trois domaines extracellulaires (α1, α2 et α3), d'une région transmembranaire et d'une région cytoplasmique. Les domaines α1 et α2 sont polymorphes. La chaîne β, ou encore β2-microglobuline, non polymorphe, s'associe spontanément à la chaîne α et est nécessaire à l'obtention d'un complexe stable. Les CMH de classe II (CMH-II) sont composés de deux chaînes α et β qui s'associent spontanément en hétérodimères. Chaque chaîne comporte deux domaines extracellulaires, (α1 et α2, β1 et β2), une région transmembranaire et d'une région cytoplasmique. Les domaines α1 et β1 sont polymorphes.

La diversité des peptides antigéniques qui peuvent s'associer à chaque allèle du CMH, ainsi que l'extrême polymorphisme du CMH, crée ainsi une très grande diversité de complexes P-CMH potentiellement reconnus par les cellules T. Par ailleurs, l'interaction entre le complexe P-CMH et le TCR est de faible affinité et il est difficile de l'observer en dehors d'un contact cellulaire qui implique des interactions multivalentes et d'autres molécules accessoires (4).

Ces propriétés rendent difficile d'une part la production de P-CMH sous une forme homogène, stable et capable de se fixer spécifiquement aux cellules T, d'autre part la mise en oeuvre de procédé moléculaires de criblage de la spécificité d'une cellule T associée à une maladie particulière.

Plusieurs méthodes ont été proposées pour produire de tels complexes P-CMH. Selon une première méthode, les domaines extracellulaires du CMH sont produits sous forme dénaturée dans la cytoplasme d' *E*. *Coli,* purifiés, repliés in vitro et enfin associés avec un peptide antigénique (5) . Selon une deuxième méthode, la stabilité des complexes P-CMH est améliorée par l'association des domaines extracellulaires α et β au sein une même chaîne protéique (6, brevet WO9640944), ou à l'aide de domaines de dimérisation (7, brevet WO9806749). De façon similaire, selon une troisième méthode, le complexe P-CMH est stabilisé en reliant le peptide antigénique lui-même à l'un des domaines du CMH au sein d'une seule chaîne protéique (8, 26, brevets W09604314, US5734023, WO9640944). Ces méthodes permettent d'obtenir des quantités non négligeable de complexes P-CMH, mais les procédés de fabrication et de purification sont relativement complexes, soit que les différents éléments doivent être associés in vitro, soit que la production mette en jeu des cellules eucaryotes. De plus, pour obtenir une liaison stable des P-CMH aux cellules T spécifiques, il est encore nécessaire de créer des oligomères de P-CMH, par exemple en utilisant l'avidine tetramérique (9), ou des domaines d'oligomérisation (WO 9803552, WO9806749). De tels composés sont utiles à détecter et à trier les cellules T spécifiques d'un antigène dans un mélange, comme par exemple le sang, ou à stimuler spécifiquement cette même population de cellules à des fin vaccinales ou immunothérapeutiques.

La structure moléculaire et la diversité des P-CMH rend également difficile le criblage de la spécificité des cellules T associées à une maladie particulière. Les procédés de production de P-CMH décrits ci-dessus sont peu adaptés à cet usage, dans la mesure ou ils ne permettent de produire, au coup par coup, qu'un petit nombre de complexes P-CMH contenant un peptide antigénique de séquence définie et un allèle particulier du CMH. La recherche de peptides antigéniques reste donc à l'heure actuelle une procédure complexe impliquant entre autres techniques, la mise en présence des cellules T avec des cellules eucaryotes présentatrices, exprimant un ou des allèles particuliers du CMH. Dans une première méthode, les peptides antigéniques candidats sont ajoutés dans l'essai sous forme de peptides synthétiques solubles (10). Dans une deuxième méthode, dite génétique (11) ils sont ajoutés sous forme d'ADN, d'ADNc ou de leurs fragments transfectées dans les cellules présentatrices. Les transfectants sont ensuite criblés pour leur capacité à stimuler les cellules T. Dans une méthode équivalente, ils sont ajoutés par l'intermédiaire de micro-organismes capables d'être phagocytés par les cellules présentatrices (12). Aucune de ces méthodes ne permet de cribler directement les complexes P-CMH d'après leur capacité à se fixer au TCR en l'absence de cellule présentatrices. Par ailleurs, elle ne permettent pas de cribler un très grand nombre de peptides.

On connaît par ailleurs dans l'art antérieur la méthode dite du "phage display", décrite par exemple dans la demande de brevet internationale PCT publiée sous le No. WO9702342. Cette méthode permet d'associer une séquence d'ADN à son produit d'expression au sein d'une même particule de phage. Cette caractéristique permet le criblage d'une grande diversité de ligands d'après leur capacité à fixer de façon stable un récepteur d'intérêt (13). Le système du phage display a été utilisé pour présenter de petits peptides d'environ 10 acides aminés à la surface des bactériophages (14). Des molécules de taille plus importantes ont été également exprimées à la surface des bactériophages, notamment des anticorps (15). Cependant, la manipulation des bactériophages en vue de la présentation de protéines de taille importante à leur surface pose de nombreux problèmes, liés à l'assemblage des protéines et à leur présentation sur l'enveloppe du phage (16), ou encore à leurs propriétés de sécrétion ou de repliement dans le périplasme bactérien (17).

Dans un premier aspect, la présente invention concerne un phage génétiquement modifié de manière à présenter un ou plusieurs complexes P-CMH à sa surface.

Dans un autre, aspect, la présente invention se rapporte à un procédé de préparation d'un phage exprimant à sa surface un complexe P-CMH, les deux éléments dudit complexe pouvant être liés de manière covalente ou non.

Plus particulièrement la présente invention envisage deux modes de réalisation du procédé. Dans un premier mode, la demanderesse envisage l'expression des complexes P-CMH sous forme soluble. Dans un second mode, elle envisage l'expression desdits complexes à la surface de phages.

Dans un autre aspect, la présente invention se rapporte également à l'utilisation de phages obtenus selon l'un ou l'autre des modes de réalisation du procédé de l'invention pour marquer, trier ou moduler l'activité de cellules T *in vitro* ou *in vivo.*

Dans un dernier aspect, la présente invention se rapporte également à un procédé d'utilisation des phages obtenus selon l'un ou l'autre des modes de réalisation du procédé de l'invention pour la préparation de banques (ou bibliothèques) de phages pour cribler et isoler des peptides antigéniques reconnus par des cellules T.

Les objets et les avantages de l'invention pourront être plus clairement appréciées à la lumière des descriptions détaillées qui suivent.

La présente invention concerne en premier lieu un phage génétiquement modifié de manière à produire un complexe P-CMH et à le présenter à sa surface. Les principaux avantages par rapport aux complexes P-CMH de l'art antérieur résident dans le fait que les complexes présentés à la surface d'un phage permettent l'accès direct à la séquence nucléotidiques codant pour les peptides et le CMH présent, puisque cette séquence est présente à l'intérieur du phage. La présente invention est donc relative tout particulièrement à un phage présentant à sa surface un ou plusieurs comlexe(s) P-CMH.

Les phages objet de la présente invention sont obtenus par l'expression du ou des complexe(s) P-CMH selon le procédé tel que décrit ci-dessous. L'invention concerne donc aussi un procédé préparation d'un phage exprimant à sa surface un ou plusieurs complexe(s) peptide-CMH caractérisé en ce que ledit procédé comprend les étapes suivantes :
a) la préparation d'une séquence nucléotidique comprenant, d'une part, une séquence codant successivement pour un peptide signal, un peptide antigénique, un peptide de liaison, un ou des domaines extracellulaires d'un ou plusieurs produits du CMH et, d'autre part, une séquence fonctionnelle, laquelle permet la présentation de la protéine de fusion codée par ladite séquence nucléotidique à la surface d'un phage, laquelle séquence fonctionnelle est choisie dans le groupe comprenant les protéines de manteau de phage,de préférence les protéines g3p ou g8p;
b) le clonage de ladite séquence nucléotidique obtenue en (a) dans un vecteur de type phagemide, l'expression de ladite séquence nucléotidique est sous la dépendance d'un promoteur du phagemide, permettant l'expression dans une bactérie de ladite séquence obtenue en (a) ;
c) la transfection d'une bactérie avec le vecteur obtenu à l'étape (b), ladite bactérie étant de préférence E. coli ;
d) l'infection desdites bactéries à l'aide d'un phage dit helper, permettant la génération de phages présentant le(s) complexe(s) peptide-CMH à leur surface ;
e) l'isolement des particules du phage à partir du surnageant de culture.

L'invention concerne également un procédé de préparation d'un phage exprimant à sa surface un ou plusieurs complexe(s) peptide-CMH caractérisé en ce que ledit procédé comprend les étapes suivantes :
a) la préparation d'une séquence nucléotidique comprenant, d'une part, une séquence codant successivement pour un peptide signal, un peptide antigénique, un peptide de liaison, un ou des domaines extracellulaires d'un ou plusieurs produits du CMH et, d'autre part, une séquence fonctionnelle, laquelle permet la présentation de la protéine de fusion codée par ladite séquence nucléotidique à la surface d'un phage, laquelle séquence fonctionnelle est choisie dans le groupe comprenant les protéines de manteau de phage,de préférence les protéines g3p ou g8p;
b) le clonage de ladite séquence nucléotidique obtenue en (a) dans un vecteur de type phage, l'expression de ladite séquence nucléotidique est sous la dépendance d'un promoteur du phage, permettant l'expression dans une bactérie de ladite séquence obtenue en (a) et la synthèse de particules de phage contenant ladite séquence nucléotidique obtenue en (a) ;
c) la transfection d'une bactérie avec le vecteur obtenu à l'étape (b), ladite bactérie étant de préférence E. coli ;
d) l'isolement des particules du phage à partir du surnageant de culture.

Par peptide signal, il faut entendre un peptide permettant l'export de la protéine de fusion dans le périplasme, où il est clivé (18). Les conditions chimiques régnant dans le périplasme sont en effet favorable au bon repliement des domaines du CMH (5). Il est possible d'utiliser les peptides signal naturels présents dans les protéines bactériennes ou les protéines du bactériophage fd g3p et g8p (19), ainsi que les peptides signal améliorés obtenus par mutagenèse et sélection appropriée (20). Il est en surtout primordial de déterminer précisément le site de clivage entre le peptide signal et le peptide antigénique. La modification, l'ajout ou la soustraction de résidus à l'extrémité N-terminale des peptides antigéniques peut en effet affecter les propriétés antigéniques du peptide (3). A cet effet, la séquence de l'ADN du peptide signal doit être modifiée de façon à insérer silencieusement un site de clonage adapté à son assemblage avec les autres éléments.

De manière avantageuse, le peptide signal utilisé est le peptide signal de la protéine pectine lyase B (PelB, 21). Ce peptide signal permet un clivage naturel de la protéine de fusion exactement devant le premier résidu du peptide antigénique.

Dans une autre forme de réalisation, la demanderesse envisage de remplacer le peptide signal par une protéine connue pour s'exprimer à haut niveau dans le périplasme du micro-organisme, comme la maltose binding protein (22). Le clivage entre cette protéine et le peptide antigénique s'effectue alors à l'aide d'un enzyme spécifique, parmi lesquels on peut citer le facteur Xa (23) ou d'autres protéases.

Par peptide antigénique la demanderesse entend aussi une diversité de peptides antigéniques.

Le peptide antigénique utilisé dans l'étape (a) peut-être codé par un fragment de séquence nucléique naturelle extraite d'un organisme, d'un tissu, d'une culture cellulaire, d'une organelle, ou d'un virus. Ces fragments peuvent être obtenus à partir de l'ADN génomique ou complémentaire de l'échantillon, par exemple par fragmentation enzymatique (24). Il est alors particulièrement avantageux que certaines séquences extraites des tissus (celles codant pour une famille de protéines, par exemple) soient spécifiquement enrichies par PCR en utilisant des amorces spécifiques. De même, certaines séquences non désirées peuvent être soustraites par hybridation selon les méthodes connues en l'état de l'art (25). Dans ce cas, il est préférable de cloner les fragments obtenus à l'aide d'adaptateurs permettant d'introduire à chaque extrémité un site de clonage adapté à leur assemblage avec les autres éléments.

Dans une autre forme de réalisation, le peptide antigénique utilisé dans l'étape (a) est codé par une séquence nucléotidique de synthèse de séquence déterminée, ou dont tout ou partie de l'enchaînement de nucléotides est aléatoire. De telles séquences sont aisément obtenues à l'aide d'appareils synthétisant des oligonucléotides. Il est alors particulièrement avantageux que l'enchaînement des nucléotides aléatoires soit de séquence NNK, ce qui diminue la probabilité d'un codon Stop. De même, les séquences partiellement aléatoires ci-dessus sont caractérisées par la présence de nucléotides fixées à certaines positions, correspondant aux résidus d'ancrage des peptides antigéniques dans leur site de liaison au CMH (3). Dans ce mode, il est particulièrement avantageux d'obtenir les fragments aléatoires par PCR en utilisant une amorce tout ou partie aléatoire, ce qui permet d'introduire les sites de clonage adaptés à leur assemblage avec les autres éléments.

Il est également avantageux que les oligonucléotides codant pour le peptide antigénique contiennent de 24 à 36 nucléotides, soit 8 à 12 codons, ce qui constitue la taille optimale des peptides liant le CMH (3).

Le peptide de liaison est un peptide flexible qui relie le peptide antigénique au premier domaine du CMH de façon covalente (8, 26). Cette caractéristique est avantageuse dans la mesure ou le CMH qui s'associe à un peptide adopte plus facilement une conformation fonctionnelle.

Le peptide de liaison comportera avantageusement entre 5 et 25 acides aminés choisis préférentiellement parmi la glycine, la serine, la thréonine, l'alanine et la proline. Il est également préférable que la séquence d'ADN qui la code comporte des sites de clonage adaptés à leur assemblage avec les autres éléments.

Par domaines extracellulaires du CMH, les inventeurs font référence au CMH de classe I et de classe II. Chez l'homme, les CMH-I sont codés par les loci HLA-A, HLA-B et HLA-C. Les CMH-II sont codés par les loci HLA-DR, HLA-DP et HLA-DQ. Les domaines extracellulaires sont les domaines α1, α2, α3 et β2-microglobuline (β2) du CMH de classe I ou bien les domaines α1, α2, β1 et β2 du CMH de classe II. Les séquences de ces domaines pouvant être polymorphes, tous leurs allèles naturels sont inclus dans la définition. Il en est de même pour les variants obtenus par mutagenèse dirigée, en une ou quelques positions. Ces mutations peuvent en effet être utile à augmenter leur production ou leur sécrétion par le micro-organisme. Les domaines du CMH, ou l'ensemble des domaines appartenant à une même chaîne naturelle du CMH peuvent être obtenus par PCR en utilisant des amorces spécifiques. De même, il peut être avantageux d'obtenir en une étape différents allèles des domaines polymorphes (par exemple ceux d'un même individu) en utilisant des amorces de PCR dégénérées. A chaque domaine peut donc se substituer une diversité de domaines. La façon dont ces domaines sont ensuite combinés entre eux dans l'étape (a) en vue de leur expression fait l'objet des différents modes suivants. Pour effectuer cette combinaison, il peut-être utile de relier les domaines les uns aux autres à l'aide de peptides flexibles (L).

Les quatre domaines sont présents dans la protéine de fusion de l'étape (a). Parmi les enchaînements préférés, on peut citer les enchaînements α1-α2-α3-L-β2 pour le CMH-I et α1-α2-L-β1-β2 ou β1-β2-L-α1-α2 pour le CMH-II, qui respectent au maximum l'enchaînement naturel des domaines. On peut également citer l'enchaînements β1-L-α1-L-β2-L-α2 du CMH-II qui est structurellement analogue à l'enchaînement α1-α2-α3-L-β2 de classe I. De même, on peut citer l'enchaînement α2-L-β2-L-α1-L-α3 de classe I qui se rapproche structurellement de l'enchaînement α1-α2-L-β1-β2 de classe II.

Dans une autre forme de réalisation, la protéine de fusion de l'étape (a) inclut les deux domaines liant le peptide antigénique, à savoir les domaines α1 et α2 du CMH-I et α1 et β2 du CMH-II. L'ajout d'un domaine α3 ou β2 de classe I ou α2 ou β2 de classe II, ainsi que d'autres domaines de la famille des immunoglobuline (D) peut-être également utile à stabiliser la protéine. Les enchaînements du type α1-α2-L-D de classe I et α1-β1-L-D ou β1-α1-L-D de classe II sont alors particulièrement avantageux.

Dans une dernière forme de réalisation, la protéine de fusion de l'étape (a) inclut seulement une chaîne du CMH, l'autre étant exprimée dans le périplasme du micro-organisme à l'aide d'un vecteur accessoire. Les enchaînement particulièrement intéressants sont alors les enchaînement naturels α1-α2-α3 ou β2 pour le CMH-I et α1-α2 ou β1-β2 pour le CMH-II ainsi que leurs analogues structuraux α1-L-α3 ou α2-L-β2 pour le CMH-I. Dans ce mode, il peut être particulièrement intéressant de favoriser l'association entre les deux chaînes du CMH dans le périplasme à l'aide de domaine accessoires, par exemple des leucine zippers (7).

Le peptide flexible L est avantageusement constitué de 5 à 25 acides aminés choisi préférentiellement parmi la glycine, la serine, la thréonine, l'alanine et la proline. Il sera également particulièrement avantageux que la séquence codant pour le peptide flexible comporte des sites de clonages permettant l'assemblage des différents domaines.

La présente invention fait aussi référence à une séquence fonctionnelle.

Ladite séquence fonctionnelle code pour une protéine qui permet la présentation de la protéine de fusion de l'étape (a) sur la paroi ou la membrane externe d'un phage. Parmi les protéines permettant la présentation de protéines de fusion à la surface du phage, on peut citer les protéines de manteau de phage, notamment les protéines de structure majeure et mineure que respectivement sont les protéines g8p (27) ou g3p (14). L'ADN codant ces protéines peut-être aisément amplifié par PCR par l'homme du métier avec des amorces spécifiques qui permettent d'introduire des sites de clonage adaptés à leur assemblage avec les autres éléments. Lors de cette opération il peut-être avantageux d'introduire en amont de la séquence des protéines d'enveloppe une séquence codant pour un peptide clivé par une enzyme protéolytique comme le facteur Xa. Le clivage de la protéine de fusion présente à la surface du phage peut être utile à obtenir à détacher les phages spécifiquement liés à un récepteur (28, 29) ou à purifier les complexes P-CMH.

Les vecteurs de l'étape (c) adaptés à la réalisation du procédé permettent l'expression de la séquence de l'étape (a). Ils doivent donc comporter les régions fonctionnelles (en particulier un promoteur, inductible ou non, et un site d'initiation de la traduction permettant l'expression de la protéine de fusion dans la bactérie. De tels vecteurs sont largement disponibles pour l'homme du métier. On peut citer par exemple les vecteurs d'E. Coli de type plasmide comme pUC19, ou de type phagemide comme puC119, pAb-tag ou pAb8, ou de type phage comme fd-DOG (40) ou fd-med. Le choix entre ces types de vecteurs est dicté par l'intérêt ou non de produire des phages ou d'empaqueter l'ADN simple brin du vecteur dans un phage. Parmi ces vecteurs, on peut avantageusement utiliser des vecteurs modifiés de façon à faire exprimer à la surface de phages d'autres molécules d'intérêt immunologique permettant par exemple la stimulation des cellules T (par exemple CD40L, CD80, CD06 ou d'autres ligands de CD28 ou CD40), leur délétion (par exemple, FasL ou d'autre ligands de CD95), ou des antigènes stimulant les cellules B.

Dans une forme préférée de réalisation du procédé, la séquence obtenue en (a) est clonée dans un vecteur du type phage, par exemple fd-med, sous la dépendance d'un promoteur du phage, qui permet à la fois l'expression dans E. Coli de la séquence obtenue en (a) et la synthèse de particules de phages contenant la séquence d'ADN obtenue en (a).

Dans une autre forme préférée de réalisation, la séquence obtenue en (a) est clonée dans un vecteur du type phagemide, par exemple dans pUC119. Ceci permet l'expression de cette séquence dans E. Coli. La production de particules de phages contenant la séquence obtenue en (a) est alors dépendante de la surinfection des même bactéries par un phage dit helper, comme VCSM13 (Stratagene). Ce mode offre l'avantage de pouvoir moduler la quantité de protéine de fusion synthétisés.

La transfection et l'induction de l'expression du produit de la séquence nucléotidique selon les étapes (c) et (d) du procédé de l'invention, peut être obtenue par tout moyen approprié (30), tel que des variations de concentration en ions ou des variations de température.

Le procédé d'expression décrit ci-dessus permet de produire dans une bactérie, des complexes P-CMH liés à une séquence fonctionnelle dans lesquels le peptide antigénique est lié de façon covalente au CMH. Néanmoins, l'homme de l'art sait que l'on peut obtenir des complexes P-CMH équivalents dans lequel le peptide antigénique et le CMH s'associent de façon non covalente, en vertu de l'affinité des peptides antigéniques pour le CMH (4). Cette association peut se produire dans le périplasme de la bactérie, ou à l'extérieur. Bien que l'obtention de complexes P-CMH non covalent soit moins simple, il permet une plus grande flexibilité dans le maniement de banques de P-CMH. Il permet en particulier de combiner une banque de peptides antigéniques à une banque d'allèles du CMH (31) ainsi que d'exprimer davantage de complexe P-CMH.

En fonction des constructions de séquences nucléotidiques et des vecteurs utilisés dans le procédé de l'invention, les Inventeurs ont obtenu des complexes P-CMH non covalents.

La présente invention envisage également l'expression de complexe P-CHH non covalents liés à une séquence fonctionnelle. L'expression est obtenue selon le procédé décrit plus haut comportant les étapes suivantes modifiées :
a') la séquence nucléotidique de l'étape (a) ne contient aucun domaine du CMH, le peptide de liaison étant directement relié à la séquence fonctionnelle,
e) Les complexes P-CMH sont formés en mettant en présence le produit d'expression produit en (d) contenant le peptide antigénique et d'un ou plusieurs produits du CMH exprimés indépendamment.

Par exprimé indépendamment, on entend par exemple que les séquences codant pour le CMH et pour la séquence obtenue en (a') sont exprimées dans la même bactérie, mais sont portées par un autre vecteur. L'association entre le peptide antigénique et le CMH s'effectue alors dans le périplasme de la bactérie. Il est également possible que les séquences codant pour le CMH soient portées par le même vecteur, mais ne soient pas exprimées sous forme d'une simple chaîne avec le peptide antigénique.

Les Inventeurs ont mis en évidence dans le cadre des travaux ayant mené à la présente invention, qu'il est possible de présenter des complexes P-CMH de taille importante à la surface de phages.

L'invention a donc encore pour objet un procédé de préparation d'un phage présentant à sa surface un complexe P-CMH, consistant à exprimer un complexe P-CMH par un procédé d'expression selon la présente invention dans lequel la séquence fonctionnelle utilisée à l'étape (a) ou (a') code pour une protéine permettant la présentation du complexe P-CMH à la surface dudit phage. De manière préférée, les protéines envisagées sont la g8p et/ou la g3p. La demanderesse envisage aussi la récupération du complexe P-CMH après l'étape (d) ou (e) par tout moyen connu de l'homme du métier.

L'invention a donc également pour objet un phage présentant à sa surface le complexe P-CMH obtenu par les procédés ci-dessus.

De tels phages peuvent notamment être utilisés dans un procédé de marquage et de tri de cellules T spécifique d'un ou plusieurs complexes P-CMH.

Un tel procédé comporte les étapes suivantes:
i') on met en présence :
   - un excès de phage présentant un complexe P-CMH obtenu par le procédé de l'invention et,
   - un "pool" de cellules T présentant à leur surface un ensemble de récepteurs
ii') on sélectionne les cellules T dont les récepteurs sont liés spécifiquement avec les phages portant le complexes P-CMH de l'étape (i') en éliminant par tout moyen approprié l'excès de phage n'étant pas lié de manière spécifique
iii') on marque les phages liés spécifiquement aux récepteurs de cellules T sélectionnées à l'étape (ii') à l'aide d'un anticorps dirigé contre le phage,
iv') on récupère par tout moyen approprié les cellules T dont les récepteurs sont liés spécifiquement au phage marqué à l'étape (iii'). On peut également trier ces cellules par tout moyen approprié

De tels phages peuvent également être utilisés pour moduler de manière spécifique l'activité de cellules T, préférentiellement sous forme immobilisée.

Il est particulièrement en effet aisé d'immobiliser des phages, par exemple sur du polystyrène auquel ils adhèrent spontanément, ou par l'intermédiaire d'anticorps anti-phage immobilisés, ou encore en utilisant des vecteurs de phages modifiés exprimant par exemple des peptides qui favorise leur adhésion à des cellules (32) ou à des matériaux (33).

L'invention se rapporte donc également à une composition diagnostique et/ou vaccinale utile pour moduler de manière spécifique l'activité de cellules T comprenant un phage présentant un complexe P-CMH à sa surface.

D'une manière originale, le deuxième mode de réalisation du procédé est particulièrement apte à être utilisé pour la constitution de banques de phages exprimant à leur surface un complexe P-CMH.

Par banques de phages, on entend une diversité de phages obtenus par le procédé de l'invention en utilisant dans l'étape (a) ou (a') une séquence codant pour une diversité de peptides antigéniques.

Ces banques de phages sont utiles pour le criblage des peptides antigéniques à l'aide un récepteur de cellules T, présenté sous forme soluble ou à la surface de cellules.

La présente invention, porte donc aussi sur l'utilisation de banques de phages décrites ci-dessus pour le criblage de peptides antigéniques reconnus par un récepteur de cellule T donné.

Un procédé de criblage selon la présente invention est caractérisé en ce qu'il comporte les étapes suivantes :
i") on met en présence une banque de phages selon la présente invention et un récepteur de cellules T défini, sous forme soluble ou disposé à la surface de cellules,
ii") on sélectionne les phages présentant le complexe P-CMH liés de manière spécifique avec le récepteur T de l'étape (i") en éliminant les phages qui ne sont pas liés de manière spécifique par tout moyen approprié, tel qu'un lavage,
iii") on identifie dans les phages sélectionnés à l'étape (ii") la séquence nucléotidique codant pour le peptide antigénique.

Il est aisé pour l'homme du métier de récupérer la séquence nucléotidique d'un peptide provenant d'une banque de peptides. Il lui suffit, par exemple, à partir de la construction utilisée en (a) ou (a') d'obtenir la séquence nucléotidique incluse dans ladite construction et codant pour le peptide antigénique.

D'autres caractéristiques et avantages de la présente invention apparaîtront dans la description détaillée qui suit et qui se réfère aux dessins en annexes dans lesquels :

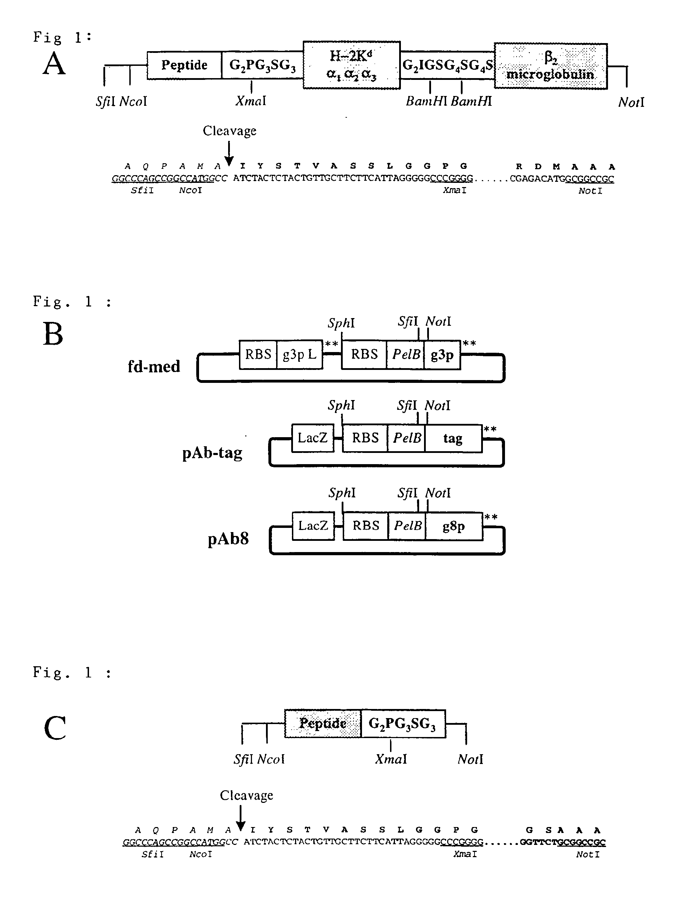

- La Figure 1 représente différents exemples d'assemblage de séquences permettant d'exprimer des complexes P-CMH. Dans les figures et les exemples qui suivent, K^{d} est le domaine d'un produit du CMH (H-2K^{d} murin) sur lequel les travaux des Inventeurs ont été effectués.
   A : L'ADN codant pour le peptide antigénique, le peptide de liaison, les domaines extracellulaires α1, α2 et α3 de K^{d} et la β2-microglobuline murine ont été assemblés par PCR.
   B : Les complexes P-CMH covalents ont été exprimés sous la forme d'une fusion avec le peptide signal de la protéine pectine lyase B (PelB) et avec des séquences fonctionnelles différentes selon le vecteur de clonage utilisé. Dans le vecteur de type phage fd-med, la séquence fonctionnelle code pour la protéine de surface mineure du phage (g3p) ; dans le vecteur de type phagemide pAb-tag, la séquence fonctionnelle code pour un peptide étiquette c-myc-1 (tag) et un codon stop(*) ; dans le vecteur du type phagemide pAb8, la séquence fonctionnelle code pour la protéine majeure de surface du phage (g8p). Dans ce vecteur, différents arrangements des domaines du CMH ont été exprimés.
   C : La séquence codant pour le peptide antigénique a été également cloné en fusion avec les séquences codant pour le peptide signal PelB, le peptide de liaison, et avec différentes séquences fonctionnelles. Les complexes P-CMH non covalents ont été réalisée par incubation avec des molécules de K^{d} produites indépendamment.
   RBS : site d'interaction du Ribosome , LacZ : séquence du promoteur inductible du gène Lac ; g3p L: peptide signal naturel de la protéine g3p de fd qui, dans le génome de fd-med est interrompu par la présence de 2 codons Stop (*). La protéine mature attendue est indiquée en caractère gras.

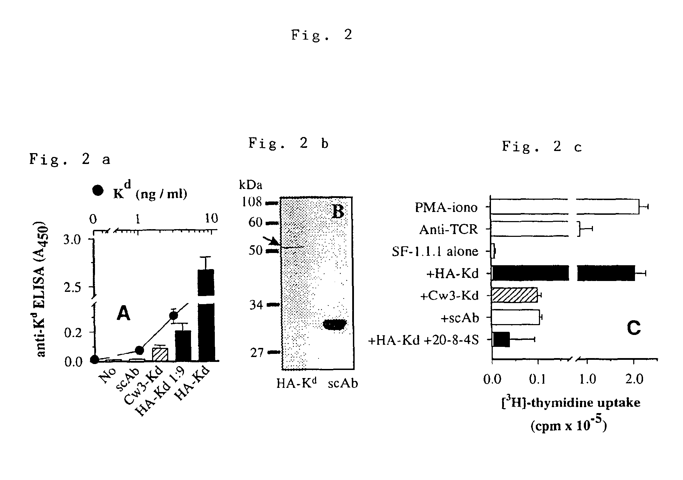

- La figure 2 représente la caractérisation de protéines de fusion P-MHC dans l'espace périplasmique.
   En A : sandwich ELISA montrant la présence de molécules P-K^{d} solubles dans l'espace périplasmique de E. Coli transformées avec les vecteurs décrits dans la figure 1. Des extraits périplasmiques (abscisse) ou les dilutions contrôles de molécules de Kd produites par un autre procédé (ronds pleins) sont incubés dans des puits de plastique recouverts par l'anticorps monoclonal 20.8.4S (dirigé contre les domaines α1/α2 de K^{d}) et révélés à l'aide d'un anticorps biotiné SF-1.1.1 (dirigé contre le domaine α3 de K^{d}), cet anticorps étant lui-même révélé par un anticorps anti-biotine conjugué à la peroxydase.
   En B : analyse par Western blot des protéines périplasmiques révélées avec un anticorps anti-c-myc-1 conjugué à la peroxydase. scAb : anticorps simple chaîne contrôle OKT3.
   En C : stimulation de cellules T spécifiques du peptide HA et restreintes à K^{d} à l'aide de molécules solubles P-K^{d} (2 ng / ml) immobilisées sur le plastique en utilisant de l'avidine et un anticorps biotiné SF-1.1.1. PMA-Iono: 10 ng/ml phorbol 12- myristate 13- acétate, 150 ng/ml ionomycine ; anti-TCR : anticorps anti-TCR biotinés (1 µg/ml) résultats exprimés sous la forme de la valeur moyenne de 3 valeurs avec déviation standard.

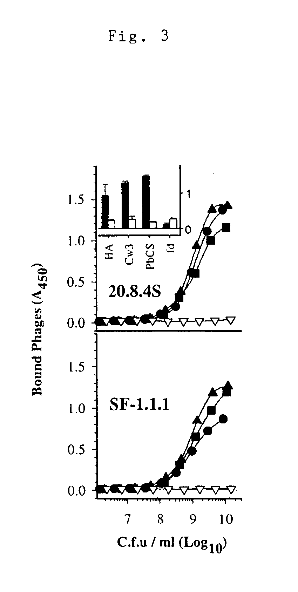

- La figure 3 représente l'expression de complexes P-CMH à la surface de phages.
   ELISA montrant l'interaction de différentes dilutions (abscisse) de phages Kd-fd exprimant le peptide HA (ronds pleins), Cw3 (triangle pleins) ou PbCS (carres pleins) ou bien l'interaction du phage sauvage fd (triangles vides) avec des puits en plastique recouverts avec l'anticorps anti-K^{d} 20.8.4.S (dirigé contre les domaines α1 et α2, cadre supérieur) ou bien avec l'anticorps SF-1.1.1 (dirigé contre le domaine α3, cadre inférieur). En insert: inhibition de l'interaction de fd et de K^{d}-fd (abscisse) en présence (barre creuse) d'un excès d'anticorps solubles 20.8.4S (moyenne ± déviation standard, n=3).

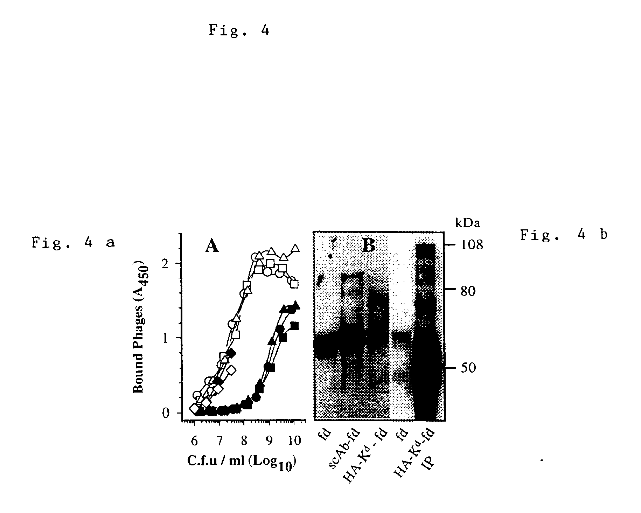

- La figure 4 représente la caractérisation biochimique de complexes P-MHC exprimés à la surface de phages.
   En A : ELISA montrant l'interaction de plusieurs dilutions (abscisse) de phages K^{d}-fd exprimant les peptides HA (carres), Cw3 (ronds) ou PbCS (triangles) avec les anticorps immobilisés sur le plastique 20.8.4S (anti-K^{d} symbole plein) ou avec l'anticorps 57D1 (anti-g3p N-terminal, symbole vide) comme contrôle les phages Cw3-K^{d}-fd ont été immunopurifiés sur un anticorps anti-K^{d} SF-1.1.1 avant la réalisation du test (losanges).
   En B : analyse par SDS-PAGE 12% des protéines du phage sauvage fd, du phage exprimant l'anticorps simple chaîne contrôle OKT3 (scAb-fd) et les phages HA-K^{d}-fd (environ 3.10⁹ c.f.u.). Les Westerns blots ont été révélés avec un anticorps g3p suivit d'un anticorps anti-souris conjugué à la peroxydase et chimiluminescence. Dans l'échantillon de phage immunopurifié (IP) la chaîne lourde de l'anticorps monoclonal SF-1.1.1 est visible sous la forme d'une large bande de masse moléculaire de 50 Kd.

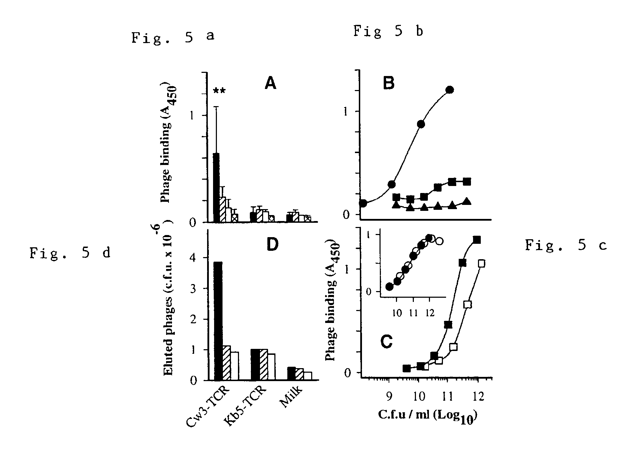

- La figure 5 illustre la spécificité de l'interaction de complexes P-MHC présentés à la surface de phages un TCR.
   En A: interaction (ELISA) du phage sauvage fd (barre doublement hachurée) ou bien du phage P-K^{d}-fd exprimant le peptide Cw3 (barre pleine), le peptide HA (barre hachurée) ou le peptide PbCs (barre vide) avec le TCR recombinant simple chaîne Cw3-1.1 (3 µg/ml, spécifique de Cw3, restreint à K^{d}, moyenne plus ou moins déviation standard, n=9), le TCR soluble Kb5-C20 (3 µg/ml TCR alloréactif anti-K^{b}, n=3), ou bien des protéines du lait (n=6). ** : L'interaction des phages Cw3-K^{d}-fd avec le TCR Cw3-1.1 est significativement plus importante (p<0.001) que celle du phage HA-K^{d}-fd, et également plus importante que celle du phage Cw3-K^{d}-fd au TCR Kb5-C20.
   En B : Analyse comparative de l'interaction (ELISA) des différentes dilutions (abscisse) de Cw3-K^{d}-fd phage avec l'anticorps monoclonal 20.8.4.S immobilisé sur du plastique (1 µg / ml, anticorps anti-K^{d} domaine α1 / α2, rond), le TCR Cw3-1.1 (1 µg/ml, carre) ou le TCR Kb5-C20 (1 µg / ml, triangles).
   En C : Analyse comparative de l'interaction (ELISA) de phage P-K^{d}-fd exprimant le peptide Cw3 (symboles pleins) ou bien le peptide HA (symboles vides) avec le TCR Cw3-1.1 immobilisé sur du plastique (à la concentration de 10 µg / ml, carrés). Les titres de phages Cw3-K^{d}-fd (abscisse) ont été corrigés (x 2.1) pour permettre de comparer à égalité sa réactivité envers l'anticorps anti-K^{d} 20.8.4S (1 µg / ml) avec celle du phage HA-K^{d}-fd (insert, ronds).
   En D : Récupération de phage P-K^{d}-fd exprimant le peptide Cw3 (barres pleines), ou bien le peptide HA (barres hachurées), ou bien le peptide PbCS (barres vides) après une seule étape de sélection contre le TCR Cw3-1.1. immobilisé sur le plastique (à la concentration de 10 µg/ml), ou bien après sélection contre le TCR Kb5-C20 (immobilisé à la concentration de 10 µg/ml), ou bien après sélection contre des protéines du lait immobilisées (abscisse).

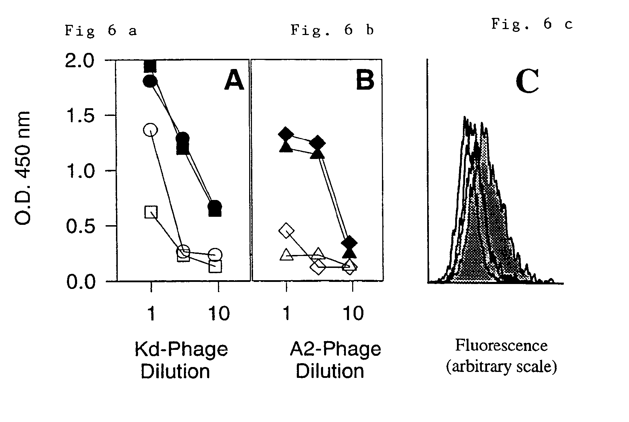

- La figure 6 représente la caractérisation de complexes P-CMH non covalents présentés à la surface de phages.
   En A: interaction (ELISA) des phages K^{d}-HA-M13 (carrés) et K^{d}-HA-fd (ronds), exprimant le complexe non covalent HA-K^{d}, avec l'anticorps anti-K^{d} 20.8.4S (symboles pleins, immobilisé à la concentration de 1 µg / ml, moyenne plus ou moins déviation standard, n=3) ou bien avec des protéines du lait (symbole vide).
   En B: interaction (ELISA) des phages A2-HA-M13 (losanges) et A2-POL-M13 (triangle) exprimant le complexe non covalent POL-A2 avec l'anticorps anti-HLA-A2 B9.12.1 (symboles pleins, immobilisé à la concentration de 1 µg / ml, moyenne plus ou moins déviation standard, n=3) ou bien des protéines du lait (symboles vides).
   En C : Marquage (FACS) des cellules 2D4 exprimant le TCR spécifique du peptide HA et restreint à K^{d} à l'aide de phages Kd-HA-M13 exprimant le complexe HA-Kd lié à la g8p (histogramme plein). Les cellules on été incubées à 4°C avec les phages, lavées, incubées avec un anticorps anti-phage biotiné, lui-même révélé par de la streptavidine marquée à la phycoérythrine. Le nombre de cellules (ordonnée) émettant la fluorescence spécifique (abscisse) est indiqué. En vide, marquage de cellules contrôles n'exprimant pas le TCR, et marquage des deux types de cellules en présence d'une inhibition par 10 µg d'anticorps anti-K^{d} 20.8.4S.

### I - Matériel et méthodes.

### 1) Constructions

Les PCR ont été réalisées dans un thermocycleur PTC100 (MJ Research) et les cycles ont été les suivants : 30 cycles (95°C 40 s, 55°C 1 min., 72°C 2 mn), 0.5 µM d'oligonucléotides, 250 mM de dNTP, 1.5 mM de MgC12 et 20 U/ml d'ADN polymérase *Taq*. Les produits de PCR ont été digérés par des enzymes de restriction obtenus chez New England Biolabs, puis purifiés par Wizard Prep PCR (Promega). Les séquences d'ADN ont été vérifiées sur les deux brins en utilisant des kits de séquences et un séquenceur automatique ABI 377 de chez Perkin-Elmer. La reverse transcriptase dérivée de l'AMV a été obtenue de Boehringer Mannheim (TITAN^{™} 1 tube RT-PCR kit).

### 2) Expression des phages

Les titres de bactériophages ont été mesurés sous la forme d'unités formant colonies (c.f.u.) en comptant le nombre de colonies d'E. Coli résistantes à l'antibiotique approprié, obtenues après infection de la souche bactérienne TG1 avec plusieurs dilutions différentes de suspension de phages.

### 3) Western Blot

Les protéines solubles et les protéines du phage ont été soumises à une analyse par électrophorèse en gel de polyacrylamide en présence de 12% de SDS (12% SDS-PAGE, Gels Novex, San Diego, Californie) dans des conditions réductrices, le contenu des gels a été ensuite transféré par électroblot sur des membranes de nitrocellulose (Hybond C extra, Amersham), les membranes ont été ensuite bloquées par l'addition d'une solution de lait écrémé dans du tampon phosphate salin (PBS) et analysées avec un anticorps monoclonal 9E10 dirigé contre l'épitope c-myc-1 (Invitrogen, dilution 1/1000 dans une solution de PBS, 1 g / 1 Tween-20, PBS-Tween) ou bien l'anticorps anti-g3p spécifiquement dirigé contre le troisième domaine de la g3p qui est nécessaire à l'incorporation de cette protéine dans la particule virale (Eurogentec, dilution 1/1000 dans une solution de PBS-Tween). Après incubation avec les anticorps les membranes ont été révélées par l'addition d'un anticorps anti-souris marqué à la peroxydase (dilution 1/1000, Sigma), ses anticorps ont été détectés par chimiluminescence (kit ECL, Amersham).

### 4) ELISA

Pour les test ELISA, des plaques à micro-puits en polystyrène (Nunc) ont été recouvertes par incubation pendant toute une nuit d'une solution de PBS contenant différents anticorps, à 4°C. Les anticorps utilisés sont l'anticorps 20.8.45 dirigé contre les domaines α1 et α2 de la molécule K^{d} (clone ATCC HB-11), l'anticorps monoclonal SF-1.1.1 dirigé contre le troisième domaine α3 de la molécule K^{d} (clone ATCC HB-159, de chez Pharmigen), l'anticorps monoclonal B-9.12.1 (de chez Immunotech), dirigé contre les molécules HLA de classe I, l'anticorps monoclonal 57D1 dirigé contre la protéine g3p du phage M13 (un don du Docteur Monaci, IRBM, Rome, Italie), et un anticorps de lapin anti-phage-fd (Sigma). Des molécules recombinantes solubles de TCR simple chaîne ont été également immobilisées à la surface de plaques ELISA. Ces TCR solubles Cw3-1.1 et Kb5-C20 ont été produits et fournis par le Docteur C. Grégoire du Centre d'Immunologie à Marseille, en France (34). Après saturation en présence d'une solution de lait écrémé dans du PBS (40 g de lait Régilait écrémé pour 1 1 de PBS) pendant 2 heures à 25°C, les phages ou les extraits périplasmiques dilués dans du PBS-lait ont été ajoutés aux plaques pendant 2 heures, puis lavés trois fois à l'aide PBS-Tween, puis lavés deux fois à l'aide de PBS. Les phages restant attachés aux protéines immobilisées sur le polystyrène ont ensuite été révélés avec un anticorps anti-M13 marqué à la peroxydase (Sigma, dilution 1/3000 dans du PBS-lait pendant 1 heure). Les molécules K^{d} solubles restant attachées aux plaques de polystyrène ont elles été révélées avec un anticorps biotiné SF-1.1.1 (Pharmingen, 1 µg/ml dans du PBS-lait) suivi par l'incubation avec un anticorps anti-biotine marqué à la peroxydase (Sigma 1/5000 dans du PBS-lait). Après trois lavages en PBS-Tween, l'activité peroxydase a été détectée par ajout du substrat TMB (Pierce).

### 5) Test de prolifération des cellules T

Pour les test de prolifération cellulaires, les cellules CD8 purifiées ont été isolées à partir de la rate et des ganglions lymphatiques de souris transgéniques pour le TCR spécifique du peptide HA et restreint à la molécule K^{d} (35). Les cellules ont été purifiées grâce à l'utilisation de l'anticorps de rat 53-6.7 dirigé contre la molécule CD8 murine (Pharmingen) par sélection positive à l'aide de billes magnétiques recouvertes d'anticorps anti-rat (Miltenyi Biotec, Bergisch-Gladbach, Allemagne). Par analyse cytofluorométrique, il fut déterminé que 90% des cellules ainsi sélectionnées étaient de phénotype CD8+ BV8+. La prolifération cellulaire des cellules purifiées (10⁵ cellules purifiées dans un puits de culture) a été mesurée après 48 heures de culture cellulaire en mesurant l'incorporation de thymidine tritiée pendant une période de 12 heures.

### II - Résultats

### 1. Construction de séquences codant pour des enchaînements de domaines du CMH

Cette méthode permet d'obtenir l'ADN codant pour différents enchaînements d'un ou plusieurs domaines du CMH. Dans cet exemple, les Inventeurs se sont intéressés à l'allèle du CMH murin H-2K^{d}. L'ADN codant successivement pour l'enchaînement α1-α2-α3-L15-β2m de K^{d} et la β2-microglobuline murine, ou L15 est un peptide de liaison de 15 aminoacides (Fig. 1A), ont été donnés par Jean-Pierre Abastado (Institut Pasteur, Paris, France, Mottez et al. 1995). Cet ADN a été amplifié par PCR en utilisant les oligonucléotides suivants : 5'-GGT GGC CCG GGG GGT GGT TCT GGG GGT GGG GGC CCA CAT TCG CTG AG et 5'-AAC AGT TTC TGC GGC CGC CAT GTC TCG ATC CCA GTA GAC G qui permettent d'ajouter des sites de clonages *Xma*I et *Not*I*.* L'ADN codant pour le CMH a été initialement cloné dans le plasmide pBluescript KS+.

Pour obtenir un enchaînement α1-α2-L18-β2m, ou L18 est un linker de 18 aminoacides, la construction précédente a été digérée avec *Bam*HI et *Bgl*II et les extrémités ont été réassociées. Alternativement, pour obtenir un enchaînement α1-α2-α3, la même construction a été digérée avec *Acc*I et *Bam*HI, les extrémités ont été traitées par le fragment de Kleenow de la polymérase en présence de dNTP, puis réassociées. Pour obtenir un enchaînement α1-α2, cette dernière construction a été digérée avec *Bam*HI et *Bgl*II et les extrémités ont été réassociées.

Une construction de contrôle codant pour un anticorps simple chaîne (scAb) a été obtenue par clonage du fragment variable simple chaîne de l'anticorps monoclonal OKT3 (36) en utilisant une procédure d'assemblage par PCR similaire.

### 2. Construction de séquences codant pour des peptides antigéniques

Cette méthode permet d'obtenir les séquences d'ADN codant pour différents peptides antigéniques. Dans cet exemple, les Inventeurs se sont intéressés au peptide antigénique HA de séquence IYSTVASSL, correspondant aux résidus [515-523] de l'hémaglutinine du virus de la grippe (37) ou bien Cw3 de séquence RYLKNGKETL, correspondant au résidu [170-179] de la molécule HLA-Cw3 (38), ou bien PbCs de séquence SYIPSAEKI correspondant au résidu [252-260] de la protéine *Circumsporozoite de Plasmodium Berghei* (39). L'ADN double brin codant pour les peptides antigéniques (Fig. 1A) a été construit par PCR en utilisant un oligonucléotide anti-sens 5'-ACC CCC CGG GCC et un oligonucléotide sens présentant la séquence générique suivante : 5'-GCC CAG CCG GCC ATG GCC-X-GGT GGC CCG GGG GGT GGT TCT GGG-3' ou X code pour le peptide antigénique. X signifie ATC TAC TCT ACT GTT GCT TCT TCA TTA pour le peptide HA, ou bien CGT TAT TTG AAA AAC GGT AAA GAA ACT TTG pour le peptide Cw3, ou bien TCT TAC ATC CCA AGC GCA GAA AAA ATA pour le peptide PbCS.

### 3. Assemblage des séquences.

Cette méthode permet l'assemblage des séquences d'ADN codant pour un peptide signal, un peptide antigénique, un peptide de liaison, un ou des domaines du CMH, et différentes séquences fonctionnelles.

Dans cet exemple, les Inventeurs se sont intéressés au peptide signal PelB qui permet le clivage de la protéine exprimée exactement avant le premier résidu N-terminal du peptide antigénique (Fig. 1A) et à un peptide de liaison de 15 aminoacides (Fig. 1A).

L'assemblage des séquences codant pour les protéines de fusion entre le peptide signal PelB, le peptide de liaison, le peptide antigénique, les domaines du CMH et la protéine g3p, à été réalisé par clonage entre les sites *Sfi*I et *Not*I dans le vecteur phage fd-med, dérivé de fd-DOG (40), qui porte le gène de résistance à la tétracycline et a été fourni par le Docteur I. Fisch de l'école Polytechnique Fédérale à Lausanne en Suisse. Le vecteur fd-med présente les avantages d'introduire un codon stop dans le gène codant pour g3p, en l'absence d'ADN complémentaire inséré. De cette manière, les cellules transformées avec fd-med ne produisent que de très faible titres de phages (moins de 10⁶ c.f.u. par litre de culture). Les vecteurs obtenus, P-CMH-fd, permettent l'expression de phages présentant des complexes P-CMH covalents liés à la g3p. Il ont été électroporés dans des bactéries de souche DH10B fournies par la compagnie Gibco.

L'assemblage des séquences codant pour les protéines de fusion avec la protéine g8p du phage au lieu de la g3p a été réalisé par clonage entre les sites SfiI et *Not*I du vecteur pAb8, qui porte le gène de résistance à l'ampicilline. Ce vecteur a été obtenu en amplifiant la séquence codant la g8p du phage par PCR en utilisant le phage fd comme matrice et les amorces sens 5'-GAT TCT AGA GGT TCG GCC GCA GCT GAG GGT GAC GAT CCC GCA et anti-sens 5'-AGT GAA TTC TTA TTA GCT TGC TTT CGA GGT GAA TTT, puis en clonant le fragment obtenu à la place du fragment NotI-EcoRI de pAb-tag. Les vecteurs finalement obtenu, P-CMH-pAb8, permettent l'expression, à l'aide de phage helper, de phages présentant des complexe P-CMH covalents liés à la g8p. Il ont été électroporés dans des bactéries de souche TG1.

L'assemblage des séquences codant pour les protéines de fusion entre le peptide signal PelB, le peptide de liaison, le peptide antigénique, les domaines du CMH et un peptide étiquette, a été réalisé par clonage entre les sites SfiI et *Not*I du vecteur pAb-tag, un dérivé du vecteur pUC119 (40) qui porte le gène de résistance à l'ampicilline, et qui été fourni par le Docteur G. Winter, MRC, Cambridge, Grande-Bretagne. Les vecteurs obtenus, P-CMH-pAb-tag permettent l'expression des protéines P-CMH solubles après induction du promoteur LacZ. Ils ont été électroporés dans des bactéries de souche DH10B fournies par la compagnie Gibco.

### 4. Assemblage de séquences permettant la production de complexes P-CMH non covalents.

Cette méthode permet de transformer les séquences d'ADN obtenues dans l'exemple 3 en vue de la production de complexes P-CMH non covalents. Dans cet exemple, les Inventeurs se sont intéressés à transformer le vecteur HA-K^{d}-pAb-tag en reliant directement le peptide de liaison au site *Not*I du vecteur pAb-tag (Fig. 1C). Un fragment de PCR a été obtenu en utilisant le vecteur pAb-tag comme matrice et les oligonucléotide sens 5'-CAG GAA ACA GCT ATG ACC A et anti-sens 5'-AGC TGC GGC CGC CCC ACC CCC AGA ACC. Ce fragment a été inséré dans le vecteur HA-Kd-pAb-tag entre les sites *Sph*I et *Not*I pour obtenir le vecteur HA-pAb-tag, qui permet d'exprimer une fusion entre le peptide antigénique, un peptide de liaison de 15 aminoacides, et un peptide étiquette sous forme soluble. La même transformation a été appliquée à différents vecteurs P-CMH-pAb8 et P-CMH-fd.

### 5. Construction de séquences codant pour une diversité aléatoire de peptides antigéniques

Cette méthode permet d'exprimer la totalité des variants désiré d'un peptide antigénique. Dans cet exemple, les Inventeurs ont cloné dans un premier cas les variants du peptide antigénique HA aux positions 4 et 6 (de séquence IYSTxVxSSL ou x représente n'importe quel aminoacide), et dans un second cas tous les variants conservant les résidus d'ancrage à la molécule K^{d} (de séquence xYxxxxxxxL). L'ADN codant pour ces diversités de peptides antigéniques a été construit par PCR en utilisant comme matrice le vecteur pAb-tag, un oligonucléotide sens 5'-CAG GAA ACA GCT ATG ACC A et un oligonuléotide anti-sens présentant la séquence générique suivante : 5'- ACC CCC CGG GCC ACC X GGC CAT GGC CGG CTG GGC CGC, ou X code pour une diversité de peptides antigéniques. X signifie TAA TGA AGA MNN AAC MNN AGA GTA GAT dans le premier cas, et TAA MNN MNN MNN MNN MNN MNN GTA MNN dans le second cas. Les fragments obtenus ont été digérés avec *Sph*I et *Xma*I et clonés aux mêmes sites dans les vecteurs décrits Figure 1B. Le séquençage de 36 fragments obtenus a montré la diversité attendue aux positions variables. Cette méthode a également été utilisée pour cloner le peptide antigénique POL, dérivé du gène *pol* du virus VIH, de séquence ILKEPVHGV à l'aide d'un oligonucléotide ou X signifie CAC GCC GTG CAC TGG CTC TTT AAG GAT.

### 6. Construction de séquences codant pour une diversité de peptides antigéniques dérivés d'un tissus

Cette méthode permet d'exprimer la totalité des différents peptidiques d'une longueur comprise entre 8 et 12 aminoacides pouvant dériver d'une protéine particulière. Dans cet exemple, les peptides sont issus des variants de la protéase du virus VIH-1 isolés du sang d'un patient.

L'ARN total a été extrait à partir de 1 ml de sang de patient contenant des cellules infectées selon Sambrook (30). L'ADNc correspondant aux transcrits de la protéase de VIH a été amplifié par reverse transcription suivie d'une PCR (RT-PCR) en une seule étape, à l'aide des amorces 5'-AGA GCT TCA GGT TTG GGG et 5'-AAG CCA GGA ATG GAT GGC. Les séquences spécifiques ont été à nouveau amplifiées par PCR avec les amorces 5'-GAA GCA GGA GCC GAT AGA CA et 5'-CCT ATT GAA ACT GTA CCA GT. 100 µg du produit de PCR ont été fragmentés par digestion contrôlée à la DNAse-I (30). Les fragments mesurant environ 30 paires de bases ont été purifiés par gel d'agarose, purifiés, traités par la T4 DNA polymérase et le fragment Klenow de la DNA polymérase I, associés à un adaptateur à bouts francs spécifiquement conçu permettant de les cloner dans le site NaeI du vecteur pAb-tag. Le séquençage de 36 fragments obtenus a montré que 30 fragments avaient 24 à 36 paires de base et codaient pour la protéase du VIH.

### 7. Expression de complexes P-CMH covalents solubles et fonctionnels dans le périplasme.

Cette méthode permet d'exprimer des complexes P-CMH covalents dans le périplasme d' *E. Coli* et de vérifier qu'ils sont repliés de manière appropriée. Dans cet exemple, les Inventeurs ont exprimé des complexes P- K^{d} muni du peptide étiquette c-myc-1 et les ont caractérisés.

Les clones bactériens transfectés avec un vecteur P-CMH-pAb-tag ont été cultivés pour multiplication à 37°C dans un milieu 2YT comprenant 100 µg/l d'ampicilline, 10 g/l de glucose jusqu'à ce que la densité optique de la culture atteigne 0.6 par mesure de densité optique à 600 nm. L'expression protéique a ensuite été induite dans les bactéries par l'addition d'isopropyl-β-D-thiogalactoside (1 mM) pendant une durée de 4 heures à la température de 30 °C . Les bactéries ont été ensuite centrifugées et les culots bactériens ont été incubés pendant 45 min. à 4°C dans une solution comprenant 2 mM de Tris-HCl pH 8.0, 0.5 M sucrose, 0.5 mM EDTA. Après centrifugation (10 000 g pendant 20 min.) les extraits périplasmiques ont été filtrés (0.22 mm) et stockés à -20°C.

Les résultats montrent (Fig. 2) que des molécules recombinantes HA-K^{d} et Cw3-K^{d} sont produites et peuvent être extraites du périplasme de E. Coli après induction. D'une manière intéressante, les protéines de fusion interagissent spécifiquement avec les anticorps monoclonaux 20.8.4S et SF-1.1.1 qui reconnaissent des épitopes conformationels présents sur les domaines α1/α2 et les domaines α3 de K^{d}, respectivement (Fig. 2A). Les protéines HA-K^{d} et Cw3-K^{d} ont été ensuite quantifiées par une méthode ELISA en sandwich (environs 500 et 30 ng/l de culture bactérienne, respectivement Fig. 2A). Finalement, des molécules HA-K^{d} peuvent être détectées par Western Blot à la taille attendue (50 kDa, Fig. 2B).

Les résultats montrent également (Fig. 2C) que la conformation adoptée par le complexe P-K^{d} covalent sécrété dans le périplasme est capable d'entraîner la stimulation spécifique de cellules T comme cela a été décrit précédemment à partir de complexes P-K^{d} covalents produits dans des systèmes eucaryotes (26).

Les extraits de protéines périplasmiques contenant des molécules P-K^{d} solubles ont été immobilisées sur du plastique grâce à l'anticorps monoclonal SF-1.1.1 lui même immobilisé (1 µg / ml en PBS). Cet anticorps ne gêne pas l'interaction entre P-K^{d} et le TCR (41). Comme il est montré dans la Figure 2C, les complexes HA-K^{d} immobilisés sont capables de stimuler de manière spécifique du peptide HA des cellules T restreintes par la molécule K^{d}, alors que des quantités similaires de complexes Cw3-K^{d} immobilisés, ou bien d'un anticorps simple chaîne contrôle, n'ont aucun effet sur ces cellules mêmes T. L'activation des cellules T est inhibée d'une manière spécifique par un excès (10 µg/ml) d'anticorps monoclonal 20.8.4S.

### 8. Expression de complexes P-CMH à la surface de phages - première méthode.

Cette méthode permet d'exprimer des complexes P-CMH à la surface de phages. Dans cet exemple, les Inventeurs se sont intéressés à exprimer les complexes P-K^{d} covalents liés à la protéine g3p grâce à un vecteur de type phage (Fig. 1A, fd-med) et à caractériser les complexes obtenus.

Les clones bactériens transfectés avec un vecteur P-K^{d}-fd ont été multipliés par culture à 37°C en milieu 2YT comprenant 15 µg/ml de tétracycline, 10 g/l de glucose jusqu'à ce que la culture atteigne une densité optique de 0.9 à 600 nm, pour être ensuite maintenue en culture pendant 16 heures à 30°C. Les particules de phages ont été précipitées à partir du surnageant de culture en ajoutant à ce dernier un volume de polyéthylène glycol 8000 (Sigma, 200 mg/ml dans 2.5 M NaCl) pour 4 volumes de surnageant de culture. Le précipité a été remis en suspension dans une solution de PBS comportant 3 g/l de BSA, et filtré (0.45 µm) et stocké sous forme d'aliquot à -20°C.

Quand un mini-gène P-K^{d} est introduit dans le génome de fd-med (Fig. 1A) des titres très élevés de phages sont sécrétés dans le surnageant (de 0.1 à 5 10¹³ c.f.u. par litre de culture, dans différentes conditions de culture). Des titres comparables de phages HA-K^{d}-fd, de phages PbCs-K^{d}-fd et de phages exprimant l'anticorps simple chaîne contrôle, ont été obtenus.

La figure 3 montre que les phages P-K^{d}-fd interagissent de manière spécifique avec les anticorps monoclonaux 20.8.4S et SF-1.1.1 qui reconnaissent des épitopes conformationels présents sur les domaines α1/α2 et α3 de K^{d}, respectivement. Le niveau de bruit de fond a été défini grâce à l'utilisation du phage sauvage fd ou après compétition avec un excès d'anticorps solubles appropriés (Fig. 3, insert). Par ailleurs, les phages P-K^{d}-fd peuvent être immunopurifiés de manière efficace à l'aide d'un tube en plastique recouvert de l'anticorps monoclonal 20.8.4S dirigé contre K^{d} (utilisé à la concentration de 10 µg/ml dans 0.5 ml pour l'immobilisation). La fraction de c.f.u. retirée par immunopurification (par rapport à la quantité initialement introduite dans le tube) était respectivement de 0.25%, 0.38% pour HA-K^{d}-fd et pour Cw3-K^{d}-fd alors l'immunopurification sur des protéines de lait seul ne permettait pas d'immunopurifier plus de 0.001% de phages. D'une manière intéressante, la fraction de HA-K^{d}-fd sélectionnée après immunopurification décroît de 0.25% à 0.04% quand le phage a été produit à 37°C au lieu de 30°C.

Le nombre de molécules fonctionnelles P-K^{d} présenté par phages infectieux a été déterminé grâce à 3 différents types d'expériences.
a- La concentration des molécules K^{d} correctement repliée à été déterminée dans les suspensions de phages par un test de sandwich ELISA, comme dans le cas de la molécule K^{d} soluble (110 et 17 ng/l de culture bactérienne pour HA-K^{d}-fd et CW3-K^{d}-fd, respectivement). Du point de vue de la molarité, ceci correspond à environ 0.01 et 0.015 molécules de CMH par unité infectieuse de phages, respectivement.
b- L'interaction du phage avec les anticorps monoclonaux anti-K^{d} et 57-D1, ce dernier étant spécifique de la partie N-terminale de g3p, a été comparée (Fig. 4a). Pour obtenir le même signal ELISA (c'est-à-dire le même nombre de phages attachés au plastique) il était nécessaire d'introduire 100 fois plus de phages quand ceux-ci étaient immobilisés avec n'importe lequel des anticorps anti-K^{d} (environ 10⁹ c.f.u./ml) que lorsque les anticorps anti-g3p étaient utilisés (1x10⁷ c.f.u./ml). Comme contrôle, des phages P-K^{d}-fd ayant été immunopurifiés au préalable sur des anticorps anti-K^{d} immobilisés (et qui donc expriment tous au moins une molécule K^{d} par phage), sont, dans ce cas, aussi réactifs vis-à-vis de l'anti-g3p que de l'anti-K^{d} (Fig. 4a).
c- L'immunopurification des phages K^{d}-fd sur des tubes recouverts d'anticorps anti-K^{d} 20.8.4S était environ 60 fois moins efficace que sur des tubes recouverts d'anti-g3p (0.12% contre 7.4%).

Donc, on peut estimer qu'environ 1 à 2% de phages P-K^{d}-fd présentent à leur surface au moins une molécule fonctionnelle P-K^{d}.

L'analyse par Western Blot des protéines des phages a été réalisée à l'aide d'un anticorps monoclonal qui interagit avec le domaine C-terminal de g3p (Fig. 4b). Cette analyse a révélé deux bandes principales (voir la ligne HA-K^{d}-fd) : une bande de poids moléculaire de 70 kDa indistincte de la bande observée dans la préparation de protéine du phage sauvage fd (référence 26), et une bande de masse moléculaire d'environ 76 KDa, qui pourrait résulter de la cassure de la protéine de fusion près du domaine C-terminal du domaine α3 de K^{d}. La bande attendue correspondant à la protéine de fusion complète HA-K^{d}-g3p (d'une masse moléculaire d'environ 110 KDa) est visible sur des gels étant chargés avec 30 fois plus de matériel (résultats non montrés) ou quand les phages sont au préalable immunopurifiés contre un anticorps anti-K^{d} immobilisé (voir la ligne HA-K^{d}-fd IP). Il est important de noter, que la proportion respective des bandes de 110 KDa, 76 KDa et 60 KDa au sein des préparations des phages immunopurifiés étaient d'environ 1:2:2. Une fragmentation similaire, mais moins importante, de la protéine contrôle anticorps simple chaîne fusionnée à g3p fut aussi observée, dans ce cas, une protéine de fusion complète (de taille d'environ 90 KDa), pouvait être observée sans purification préalable (voir la ligne scAb-fd dans la figure). En plus d'un clivage protéolytique possible, on peut suspecter l'existence d'un deuxième site d'interaction pour le Ribosome au sein de la séquence codant pour le domaine _3 de K^{d}. Ce site d'initiation alternatif permettant la transcription de la protéine mesurant 76 KDa pourrait être supprimé par mutagenèse dirigée.

Il était enfin très important de démontrer que les phages exprimant des complexe P-CMH covalents pouvaient se fixer spécifiquement à un TCR.

La figure 5 montre que c'est la cas, par exemple en utilisant le phage Cw3-K^{d}-fd. La figure 5a montre que les phages Cw3-K^{d}-fd interagissent avec le TCR simple chaîne spécifique de Cw3 et restreint à K^{d}. L'interaction de ce TCR Cw3-1.1 avec les phages sauvages fd, ou bien avec des phages présentant K^{d} mais des peptides "irrelevants" (HA ou PbCs) est moins bonne. Aucun des phages utilisés n'interagissent avec le TCR monochaîne KB5-C20, qui est lui restreint à la molécule H-2K^{b}, quand celui-ci est immobilisé sur le plastique à la même concentration. La figure 5B montre que des concentrations 100 fois supérieures de phages Cw3- K^{d}-fd sont nécessaires pour obtenir le même signal ELISA (c'est-à-dire la même quantité de phages immobilisés sur le plastique) en présence de TCR Cw3-1.1 (environ 10¹¹ c.f.u./ml) qu'en présence d'anticorps anti- K^{d} (environ 10⁹ c.f.u./ml).

Pour étudier l'influence du peptide antigénique présenté par les phages P-K^{d}-fd sur leur interaction avec le TCR Cw3-1.1, on a mesuré en parallèle l'interaction des phages Cw3-K^{d}-fd et les phages HA-K^{d}-fd (figure 5c). Bien que les deux types de phages recombinants sont capables d'interagir avec le TCR, 3 fois moins de c.f.u. du phage Cw3- K^{d}-fd sont nécessaires pour induire le même signal ELISA, démontrant que le peptide présenté module d'une manière spécifique l'avidité du phage pour le TCR.

Afin d'exclure l'hypothèse selon laquelle les phages Cw3-K^{d}-fd interagissent mieux que les phages HA-K^{d}-fd car ils présenteraient plus de molécule K^{d}, des courbes d'activité anti-K^{d}-fd ont été obtenues dans des expériences parallèles (Fig. 5, insert) et superposées en corrigeant d'un facteur 2.1 les titres de phages Cw3-K^{d}-fd reportés en abscisse dans la Figure 5C. Avec ou sans (résultats non montrés) cette correction, les phages Cw3-K^{d}-fd interagissent 3 à 6 fois mieux que les phages HA-K^{d}-fd avec le TCR Cw3-1.1.

### 9. Expression de complexe P-CMH à la surface de phages - deuxième méthode.

Cette méthode permet également d'exprimer des complexes P-CMH à la surface de phages. Dans cet exemple, les Inventeurs se sont intéressés à exprimer les complexes P-K^{d} covalents liés à la protéine g8p grâce à un vecteur de type phagemide (Fig. 1A, pAb8) et à caractériser les complexes obtenus.

Les clones bactériens transfectés avec le vecteur HA-K^{d}-pAb8 ont été multipliés par culture à 37°C en milieu 2YT comprenant 100 µg/ml d'ampicilline, 10 g/l de glucose jusqu'à ce que la culture atteigne une densité optique de 0.9 à 600 nm. Apres centrifugation, les bactéries ont été remises en culture pendant 1 heure à 37°C en l'absence de glucose mais en présence de 10¹¹ c.f.u. de phage helper VCSM13 (Stratagene), qui porte la résistance à la kanamycine. Apres ajout de kanamycine (50 µg / ml), la culture est prolongée sur la nuit à 30°C. Les particules de phages sont récupérées comme décrit dans l'exemple 8.

Les phages HA-K^{d}-M13 exprimant le peptide antigénique HA réalisés d'après ce protocole ont été obtenus avec un bon titre (> 10¹¹ c.f.u. / 1). Ces phages, mais pas les phages VCSM13, expriment les épitopes spécifiques de K^{d} reconnus par l'anticorps 20.8.4S ainsi qu'il a été mesuré dans un ELISA en tout point identique à celui de l'exemple 8. Les densités optiques (moyenne ± déviation standard, n = 3) observées à 450 nm sont représentées dans le tableau 1.

**Tableau 1**

| *Phage* | *Anticorps* | *Densité optique* |
|---|---|---|
| HA-K^{d}-M13 | 20.8.4S | 1.075 ±0.133 |
| | aucun | 0.020 ± 0.005 |
| VCSM13 | 20.8.4S | 0.032 ± 0.010 |
| | aucun | 0.027 ± 0.005 |

Ces résultats montrent que les complexe P-CMH exprimés par cette méthode sont présents à la surface du phage et correctement repliés.

### 10. Expression de complexes P-CMH présentant différents enchaînements des domaines du CMH.

Cette méthode permet d'exprimer des complexes P-CMH présentant différents enchaînements des domaines du CMH. Dans cet exemple, les Inventeurs se sont intéressés à exprimer des complexes HA-K^{d} formés de différents enchaînements des domaines de K^{d} liés à la protéine g8p selon le protocole de l'exemple 9 et à caractériser les complexes obtenus.

Les densités optiques (dans un ELISA en tout point identique à celui de l'exemple 9) observées à 450 nm sont représentées dans le tableau 2 (moyenne ± déviation standard, n =3):

**Tableau 2**

| *Enchaînement* | *Densité optique* |
|---|---|
| α1-α2-α3-L-β2m | 0.275 ± 0.080 |
| α1-α2-α3 | 0.682 ± 0.130 |
| α1-α2-β2 | 0.231 ± 0.051 |
| α1-α2 | 0.127 ± 0.009 |
| VCSM13 contrôle | 0.033 ± 0.026 |

Ces résultats indiquent que pour tous les enchaînements testés, les complexes P-CMH s'expriment de façon analogue à la surface du phage et sont correctement repliés.

### 11. Expression de complexe P-CMH non covalents.

Cette méthode permet d'exprimer des complexes P-CMH non covalents. Dans cet exemple, les Inventeurs se sont intéressés à exprimer dans un premier cas des complexes HA-K^{d} et POL-A2 non covalents liés à la protéine g3p ou la protéine g8p et à caractériser les complexes obtenus.

Selon le protocole de l'exemple 4, les séquences alternatives codant pour le peptide HA et le peptide de liaison (Fig. 1C) ont été assemblées dans le vecteur pAb8 pour obtenir le vecteur HA-pAb8. De même, les séquences alternatives codant pour le peptide POL et le peptide de liaison ont été assemblées dans le vecteur pAb8 pour obtenir le vecteur POL-pAb8. La même opération a été effectuée avec dans le vecteur fd-med pour obtenir les vecteurs HA-fd et POL-fd.

Dans un premier temps, les phages HA-fd et POL-fd ont été produits selon le protocole de l'exemple 8 à partir des vecteurs HA-fd et POL-fd, respectivement. De même, les phages HA-M13 et POL-M13 ont été produits selon le protocole de l'exemple 9 à partir des vecteurs HA-pAb8 et POL-pAb8 respectivement.

Dans un deuxième temps, 1 ml des solutions de phages (typiquement 10¹¹ c.f.u. / ml) obtenues ont été incubés pendant 12 heures à 25°C puis pendant 48 heures à 4°C avec de la molécule de CMH soluble produite indépendamment (1 µg / ml) puis conservé à 4°C. L'incubation a été conduite dans une chambre de dialyse munie d'une membrane perméable à moins de 10 KDa (Slide-A-Lyser, Pierce). La molécule K^{d} a été aimablement fournie par le Dr Abastado, de l'Institut Pasteur à Paris (6). La molécule HLA-A2 a été obtenue d'après la méthode de Garboczi et al (5) .

La Figure 6A montre par un test ELISA identique à celui décrit dans l'exemple 9 que les phages K^{d}-HA-M13 (exprimant le complexe non covalent HA-Kd lié à la g8p), et K^{d}-HA-fd (exprimant le complexe non covalent HA-K^{d} lié à la g3p) sont capables de se lier à un anticorps anti-K^{d} immobilisé. De même, la Figure 6B montre que les phages A2-POL-M13 (exprimant le complexe non covalent POL-A2 lié à la g8p) et A2-POL-fd (exprimant le complexe POL-A2 non covalents liés à la g3p) sont également capables de se lier à un anticorps anti-A2 immobilisé.

### 12. Méthode de criblage de peptide antigénique.

Cette méthode de criblage consiste à mettre en présence des phages exprimant une diversité de peptides antigéniques sous forme de complexe P-CMH et un TCR, à laver les phages non liés spécifiquement, à détacher les phages fixés spécifiquement, et à récupérer la séquence nucléotidique codant pour le peptide.

Les Inventeurs ont donc procédé à un cycle de sélection de phage décrits dans l'exemple 8 d'après leur capacité à lier le TCR immobilisé Cw3-1.1. Les phages (typiquement 10¹⁰ - 10¹¹ c.f.u.) ont été incubés pendant 2 heures à la température de la pièce dans des tubes en plastique ou dans des micro-puits recouverts pendant la nuit avec une solution de TCR soluble ou d'anticorps monoclonaux (1 à 10 _g/ml dans du PBS). Après 5 lavages en PBS-Tween et 2 lavages en PBS, les phages restant attachés au polystyrène ont été élués de la phase solide par traitement à l'acide (50 mM glycine-HCl, 0.75 M NaCl, pH 2.8 pendant 5 min.), l'éluat a été ensuite neutralisé par addition de Tris-HCl 1 M pH 7.4. La quantité de phages libres ou élués a été titrée de manière parallèle.

La figure 5D montre que les phages qui expriment le peptide Cw3 spécifique du TCR (Cw3-K^{d}-fd) sont enrichi 4 fois plus que les phages les phages K^{d} exprimant le peptide HA irrelevant. De plus, cet enrichissement est spécifique dans la mesure ou les phages Cw3-K^{d}-fd ne sont pas enrichis par un cycle de sélection sur un TCR KB5-C20 ou sur des protéines du lait immobilisées en phase solide. Les phages Cw3-K^{d}-fd récupérés après liaison au TCR ont pu infecter des bactéries TG1, et leur séquence nucléotidique a pu être amplifiée par PCR dans la région d'intérêt.

### 13. Méthode de marquage et de tri de cellules.

Cette méthode de marquage de cellules exprimant des TCR consiste à incuber les phages présentant un complexe P-CMH avec les cellules puis à révéler les cellules spécifiques grâce aux phages liés.

En conséquence, les Inventeurs ont mis en présence, dans un volume de 100 µl et pendant 4 heures à 4°C, les phages Kd-HA-g8p obtenus selon le protocole de l'exemple 11 (10¹⁰ c.f.u.) avec les cellules 2D4, dérivées de la lignée BW- (42) dans laquelle les gènes du TCR spécifique du peptide HA et restreint à K^{d} ont été transfectés. Les cellules ont été lavées à 4°C, puis les phages fixés aux cellules ont été révélés par 1 µg d'anticorps anti-phage biotiné puis, après un second lavage, avec 1 µg de streptavidine phycoerythrine (Sigma). La Figure 6B montre que seules les cellules 2D4 sont marquées par les phages et que ce marquage est inhibé en présence d'un excès (10 µg) d'anticorps anti-K^{d} 20-8-4S.

### 14. Méthode de stimulation des cellules T.

Cette méthode de stimulation de cellules T spécifique d'un complexe P-CMH consiste à incuber un complexe P-CMH immobilisé avec les cellules puis à révéler l'activation des cellules T spécifiques.

Dans cet exemple les Inventeurs se sont intéressé à étudier la stimulation de cellules T spécifiques du complexe HA-K^{d} par différents complexes P-K^{d} immobilisés et exprimant les variants du peptide HA en position 4 et 6 (dénommés peptides HAX46) décrits à l'exemple 5.

Les phages produits par différents clones de bactéries transféctées par le vecteur HAX46-K^{d}-pAb8 selon le protocole de l'exemple 9 ont été immobilisés sur du plastique d'une plaque à micro-puits par simple absorption. Après lavage, les cellules T spécifiques ont été distribuées et leur stimulation a été mesurée comme décrit à l'exemple 7.

On a ensuite ajouté dans les puits 50 µl d'une suspension de bactéries TG1, puis après une incubation de 1 heure à 37°C, les bactéries ont été étalées sur des plaques de cultures contenant de l'ampicilline. La séquence de l'ADN codant pour le peptide antigénique a été déterminé d'une part dans les puits donnant une stimulation supérieure à 5 10⁴ cpm (groupe positif, voir Fig. 2C), d'autre part dans les puits donnant une stimulation inférieure à 2 10⁴ cpm (groupe négatif). Sur 18 séquences obtenues dans le groupe 1, 15 codaient pour une glycine en position 6, mutation connue pour améliorer la stimulation des ces cellules T (Liblau R, Hôpital Pitié Salpétrière, Paris, observations non publiées). Sur 18 séquences obtenues dans le groupe 2, seulement une codait pour ce résidu en cette position. Ces résultats indiquent qu'il est possible d'observer l'activation spécifique des cellules T avec les complexes P-CMH immobilisé.

### REFERENCES.

1- Wilson IA, Garcia KC. Curr Opin Struct Biol 1997. Dec; 7 (6) :839-48. T-cell receptor structure and TCR complexes.
2- Jones EY. Curr Opin Immunol 1997 Feb; 9 (1) :75-9. MHC class I and class II structures.
3- Rammensee HG. Curr Opin Immunol 1995 Feb;7(1):85-96. Chemistry of peptides associated with MHC class I and class II molecules.
4- Margulies DH. Curr Opin Immunol 1997 Jun;9(3):390-5. Interactions of TCRs with MHC-peptide complexes: a quantitative basis for mechanistic models.
5- Garboczi DN, Hung DT, Wiley DC. Proc Natl Acad Sci U S A 1992 Apr 15;89(8):3429-33. HLA-A2-peptide complexes: refolding and crystallization of molecules expressed in Escherichia coli and complexed with single antigenic peptides.
6- Godeau F, Luescher IF, Ojcius DM, Saucier C, Mottez E, Cabanie L, Kourilsky P. J Biol Chem 1992 Dec 5;267(34):24223-9. Purification and ligand binding of a soluble class I major histocompatibility complex
   molecule consisting of the first three domains of H-2Kd fused to beta 2-microglobulin expressed in the baculovirus-insect cell system.
7- Scott CA, Garcia KC, Carbone FR, Wilson IA, Teyton L. J Exp Med 1996 May 1;183(5):2087-95. Role of chain pairing for the production of functional soluble IA major histocompatibility complex class II molecules.
8- Kozono H, White J, Clements J, Marrack P, Kappler J. Nature 1994 May 12;369(6476):151-4. Production of soluble MHC class II proteins with covalently bound single peptides.
9- Altman JD, Moss PAH, Goulder PJR, Barouch DH, McHeyzer-Williams MG, Bell JI, McMichael AJ, Davis MM. Science 1996 Oct 4;274(5284):94-6. Phenotypic analysis of antigen-specific T lymphocytes.
10- Hemmer B, Vergelli M, Pinilla C, Houghten R, Martin R. Immunol Today 1998 Apr;19(4):163-8. Probing degeneracy in T-cell recognition using peptide combinatorial libraries.
11- Van der Bruggen P, Traversari C, Chomez P, Lurquin C, De Plaen E, Van den Eynde B, Knuth A, Boon T. Science 1991 Dec 13;254(5038):1643-7. A gene encoding an antigen recognized by cytolytic T lymphocytes on a human melanoma.
12- Sanderson S, Campbell DJ, Shastri N. J Exp Med 1995 Dec 1;182(6):1751-7. Identification of a CD4+ T cell-stimulating antigen of pathogenic bacteria by expression cloning.
13- Rodi DJ, Makowski L. Curr Opin Biotechnol 1999 Feb;10(1):87-93. Phage-display technology--finding a needle in a vast molecular haystack.
14- Smith GP. Science 1985 Jun 14;228(4705):1315-7. Filamentous fusion phage: novel expression vectors that display cloned antigens on the virion surface.
15- McCafferty J, Griffiths AD, Winter G, Chiswell DJ. Nature 1990 Dec 6; 348 (6301) : 552-4. Phage antibodies: filamentous phage displaying antibody variable domains.
16- Dunn IS. Curr Opin Biotechnol 1996 Oct;7(5):547-53. Phage display of proteins.
17- Bothmann H, Pluckthun A. Nat Biotechnol 1998 Apr;16(4):376-80. Selection for a periplasmic factor improving phage display and functional periplasmic expression.
18- Nielsen H, Engelbrecht J, Brunak S, von Heijne G. Protein Eng 1997 Jan;10(1):1-6. Identification of prokaryotic and eukaryotic signal peptides and prediction of their cleavage sites.
19- Beck E, Sommer R, Auerswald EA, Kurz C, Zink B, Osterburg G, Schaller H, Sugimoto K, Sugisaki H, Okamoto T, Takanami M. Nucleotide sequence of bacteriophage fd DNA. Nucleic Acids Res 1978. Dec;5(12):4495-503.
20- Schneider G, Wrede P. Biophys J 1994 Feb;66(2 Pt 1):335-44. The rational design of amino acid sequences by artificial neural networks and simulated molecular evolution: de novo design of an idealized leader peptidase cleavage site.
21- Keen NT, Tamaki S. J Bacteriol 1986 Nov;168(2):595-606. Structure of two pectate lyase genes from Erwinia chrysanthemi EC16 and their high-level expression in Escherichia coli.
22- Betton JM, Hofnung M. EMBO J 1994 Mar 1;13(S):1226-34. In vivo assembly of active maltose binding protein from independently exported protein fragments.
23- Maina CV, Riggs PD, Grandea AG 3d, Slatko BE, Moran LS, Tagliamonte JA, McReynolds LA, Guan CD. Gene 1988 Dec 30;74(2):365-73. An Escherichia coli vector to express and purify foreign proteins by fusion to and separation from maltose-binding protein.
24- Petersen G, Song D, Hugle-Dorr B, Oldenburg I, Bautz EK. Mol Gen Genet 1995 Dec 10;249(4):425-31. Mapping of linear epitopes recognized by monoclonal antibodies with gene-fragment phage display libraries.
25- Rubenstein JL, Brice AE, Ciaranello RD, Denney D, Porteus MH, Usdin TB. Nucleic Acids Res 1990 Aug 25;18(16):4833-42. Subtractive hybridization system using single-stranded phagemids with directional inserts.
26- Mottez E, Langlade-Demoyen P, Gournier H, Martinon F, Maryanski J, Kourilsky P, Abastado JP
   J Exp Med 1995 Feb 1;181(2):493-502. Cells expressing a major histocompatibility complex class I molecule with a single covalently bound peptide are highly immunogenic.
27- Greenwood J, Willis AE, Perham RN. J Mol Biol 1991 Aug 20;220(4):821-7. Multiple display of foreign peptides on a filamentous bacteriophage. Peptides from Plasmodium falciparum circumsporozoite protein as antigens.
28- Matthews DJ, Wells JA. Science 1993 May 21;260(5111):1113-7. Substrate phage: selection of protease substrates by monovalent phage display.
29- Nakayama GR, Valkirs G, McGrath D, Huse WD. Immunotechnology 1996 Sep;2(3):197-207. Improving the copy numbers of antibody fragments expressed on the major coat protein of bacteriophage M13.
30- Sambrook et al. 1989. Molecular Cloning, A Laboratory Manual.
31- Fisch I, Kontermann RE, Finnern R, Hartley O, Soler-Gonzalez AS, Griffiths AD, Winter G. Proc Natl Acad Sci U S A 1996 Jul 23;93(15):7761-6. A strategy of exon shuffling for making large peptide repertoires displayed on filamentous bacteriophage.
32- Hart SL, Knight AM, Harbottle RP, Mistry A, Hunger HD, Cutler DF, Williamson R, Coutelle C. J Biol Chem 1994 Apr 29;269(17):12468-74. Cell binding and internalization by filamentous phage displaying a cyclic Arg-Gly-Asp-containing peptide.
33- Adey NB, Mataragnon AH, Rider JE, Carter JM, Kay BK. Gene 1995 Apr 14;156(1):27-31. Characterization of phage that bind plastic from phage-displayed random peptide libraries.
34- Gregoire C, Malissen B, Mazza G. Eur J Immunol 1996 Oct;26(10):2410-6. Characterization of T cell receptor single-chain Fv fragments secreted by myeloma cells.
35- Morgan DJ, Liblau R, Scott B, Fleck S, McDevitt HO, Sarvetnick N, Lo D, Sherman LA. J Immunol 1996 Aug 1;157(3):978-83. CD8(+) T cell-mediated spontaneous diabetes in neonatal mice.
36- Arakawa F, Kuroki M, Kuwahara M, Senba T, Ozaki H, Matsuoka Y, Misumi Y, Kanda H, Watanabe T J Biochem (Tokyo) 1996 Sep;120(3):657-62. Cloning and sequencing of the VH and V kappa genes of an anti-CD3 monoclonal antibody, and construction of a mouse/human chimeric antibody.
37- Braciale TJ, Braciale VL, Winkler M, Stroynowski I, Hood L, Sambrook J, Gething MJ. J Exp Med 1987 Sep 1; 166(3): 678-92. On the role of the transmembrane anchor sequence of influenza hemagglutinin in target cell recognition by class I MHC-restricted, hemagglutinin-specific cytolytic T lymphocytes.
38- Casanova JL, Cerottini JC, Matthes M, Necker A, Gournier H, Barra C, Widmann C, MacDonald HR, Lemonnier F, Malissen B, et al. J Exp Med 1992 Aug 1;176(2):439-47. H-2-restricted cytolytic T lymphocytes specific for HLA display T cell receptors of limited diversity.
39- Romero P, Eberl G, Casanova JL, Cordey AS, Widmann C, Luescher IF, Corradin G, Maryanski JL. J Immunol 1992 Mar 15;148(6):1871-8. Immunization with synthetic peptides containing a defined malaria epitope induces a highly diverse cytotoxic T lymphocyte response. Evidence that two peptide residues are buried in the MHC molecule.
40- Hoogenboom HR, Griffiths AD, Johnson KS, Chiswell DJ, Hudson P, Winter G. Nucleic Acids Res 1991 Aug 11;19(15):4133-7. Multi-subunit proteins on the surface of filamentous phage: methodologies for displaying antibody (Fab) heavy and light chains.
41- Abastado JP, Lone YC, Casrouge A, Boulot G, Kourilsky P. J Exp Med 1995 Aug 1;182(2):439-47. Dimerization of soluble major histocompatibility complex-peptide complexes is sufficient for activation of T cell hybridoma and induction of unresponsiveness.
42- Donnadieu E, Trautmann A, Malissen M, Trucy J, Malissen B, Vivier E. J Biol Chem 1994 Dec 30;269(52):32828-34. Reconstitution of CD3 zeta coupling to calcium mobilization via genetic complementation.

## Revendications

1. Phage génétiquement modifié **caractérisé en ce qu'**il exprime à sa surface un ou plusieurs complexe(s) Peptide-CMH.

2. Procédé de préparation d'un phage exprimant à sa surface un ou plusieurs complexe(s) peptide-CMH **caractérisé en ce que** ledit procédé comprend les étapes suivantes :
a) la préparation d'une séquence nucléotidique comprenant, d'une part, une séquence codant successivement pour un peptide signal, un peptide antigénique, un peptide de liaison, un ou des domaines extracellulaires d'un ou plusieurs produits du CMH et, d'autre part, une séquence fonctionnelle, laquelle permet la présentation de la protéine de fusion codée par ladite séquence nucléotidique à la surface d'un phage, laquelle séquence fonctionnelle est choisie dans le groupe comprenant les protéines de manteau de phage,de préférence les protéines g3p ou g8p;
b) le clonage de ladite séquence nucléotidique obtenue en (a) dans un vecteur de type phage, l'expression de ladite séquence nucléotidique est sous la dépendance d'un promoteur du phage, permettant l'expression dans une bactérie de ladite séquence obtenue en (a) et la synthèse de particules de phage contenant ladite séquence nucléotidique obtenue en (a) ;
c) la transfection d'une bactérie avec le vecteur obtenu à l'étape (b), ladite bactérie étant de préférence E. coli ;
d) l'isolement des particules du phage à partir du surnageant de culture.

3. Procédé selon la revendication 2 **caractérisé en ce que** le vecteur de type phage de l'étape (b) est le phage fd-med

4. Procédé de préparation d'un phage exprimant à sa surface un ou plusieurs complexe(s) peptide-CMH **caractérisé en ce que** ledit procédé comprend les étapes suivantes :
a) la préparation d'une séquence nucléotidique comprenant, d'une part, une séquence codant successivement pour un peptide signal, un peptide antigénique, un peptide de liaison, un ou des domaines extracellulaires d'un ou plusieurs produits du CMH et, d'autre part, une séquence fonctionnelle, laquelle permet la présentation de la protéine de fusion codée par ladite séquence nucléotidique à la surface d'un phage, laquelle séquence fonctionnelle est choisie dans le groupe comprenant les protéines de manteau de phage,de préférence les protéines g3p ou g8p;
b) le clonage de ladite séquence nucléotidique obtenue en (a) dans un vecteur de type phagemide, l'expression de ladite séquence nucléotidique est sous la dépendance d'un promoteur du phagemide, permettant l'expression dans une bactérie de ladite séquence obtenue en (a) ;
c) la transfection d'une bactérie avec le vecteur obtenu à l'étape (b), ladite bactérie étant de préférence E. coli ;
d) l'infection desdites bactéries à l'aide d'un phage dit helper, permettant la génération de phages présentant le(s) complexe(s) peptide-CMH à leur surface ;
e) l'isolement des particules du phage à partir du surnageant de culture.

5. Procédé selon la revendication 4, **caractérisé en ce que** le vecteur de type phagemide de l'étape (b) est le phagemide pUC119.

6. Procédé selon l'une des revendications 4 ou 5 **caractérisé en ce que** le phage dit helper est le phage VCSM13.

7. Procédé selon l'une quelconque des revendications 2 à 6, **caractérisé en ce que** l'étape d'isolement des particules du phage à partir du surnageant de culture est faite selon les étapes suivantes :
(i) la précipitation des particules de phage à partir du surnageant de culture ;
(ii) la remise en suspension du précipité contenant lesdites particules de phage dans une solution adaptée.

8. Procédé de préparation d'un phage exprimant à sa surface un ou plusieurs complexe(s) peptide-CMH selon l'une quelconque des revendications 2 à 7 **caractérisée en ce que** ledit procédé comprend une étape (g) de purification des phages exprimant à leur surface le(s) complexe(s) peptide-CMH, de préférence par immunoprécipitation à l'aide d'anticorps spécifiquement dirigés contre le(s) complexe(s) peptide-CMH.

9. Procédé selon l'une quelconque des revendication 4 et 8, **caractérisé en ce que** l'expression dudit phagemide se fait sous la dépendance d'un promoteur inductible, de préférence le promoteur inductible du gène Lac.

10. Procédé de préparation d'un phage exprimant à sa surface un ou plusieurs complexe(s) peptide-CMH selon l'une quelconque des revendications 2 à 9, **caractérisé en ce que** le(s)dit(s) complexe(s) peptide-CMH sont associés de façon non covalente et **en ce que** :
(i) la séquence nucléotidique préparée lors de l'étape (a) ne contient aucune séquence codant pour des domaines du CMH, la séquence du peptide de liaison étant directement reliée à la séquence fonctionnelle ;
(ii) la séquence codant pour un ou plusieurs produits du CMH est soit (i) clonée dans le vecteur dans lequel le clonage de l'étape (b) est effectué, tout en étant dissociée de la séquence nucléotidique préparée lors de l'étape (a), soit (ii) clonée dans un autre vecteur d'expression ;
(iii) le ou les vecteurs sont transfectés dans une même bactérie conformément à l'étape (c).

11. Utilisation d'un phage selon la revendication 1 pour le marquage et le tri de cellules T spécifiques d'un ou plusieurs complexes Peptide-CMH.

12. Procédé de marquage et de tri de cellules T spécifiques d'un ou plusieurs complexes Peptide-CMH, **caractérisé en ce qu'**il comporte les étapes suivantes :
i) on met en présence :
un excès de phage présentant à leur surface un complexe Peptide-CMH tel que défini à la revendication 1,
un "pool" de cellules T présentant à leur surface un ensemble de récepteurs,
ii) on sélectionne les cellules T dont les récepteurs sont liés spécifiquement avec les phages portant les complexes Peptide-CMH en éliminant par tout moyen approprié l'excès de phages n'étant pas lié de manière spécifique,
iii) on marque les phages liés spécifiquement aux récepteurs de cellules T sélectionnées à l'étape (ii) à l'aide d'anticorps spécifique dudit phage,
iv) on récupère par tout moyen approprié les cellules T dont les récepteurs sont liés spécifiquement au phage marqué à l'étape (iii).

13. Composition pharmaceutique comprenant un phage selon la revendication 1.

14. Banque de phages tels que défini à la revendication 1.

15. Utilisation d'une banque de phages selon la revendication 14 pour le criblage de peptides antigéniques à l'aide d'un récepteur de cellules T (TCR) donné.

16. Procédé de criblage de peptides antigéniques, **caractérisé en ce qu'**il comporte les étapes suivantes :
i) on met en présence une banque de phages selon la revendication 14, et un récepteur de cellules T (TCR) défini, sous forme soluble ou à la surface de cellules T,
ii) on sélectionne les phages présentant le complexe Peptide-CMFi lié de manière spécifique avec le récepteur de cellule T (TCR) en éliminant les phages qui ne sont pas liés de manière spécifique,
iii) on identifie dans les phages sélectionnés à l'étape (ii) la séquence nucléotidique codant pour le peptide antigénique.

## Claims

1. A genetically modified phage, **characterized in that** it expresses one or more peptide-Major Histocompatibility Complex (peptide-MHC) complexes at its surface.

2. A method of preparing a phage expressing one or more peptide-MHC complexes at its surface, said method being **characterized in that** it comprises the following steps:
a) preparing a nucleotide sequence comprising firstly a sequence coding successively for a signal peptide, an antigenic peptide, a linker peptide, and one or more extracellular domains of one or more products of the MHC, and, secondly a functional sequence which makes it possible to present the fusion protein as coded by said nucleotide sequence at the surface of a phage, and which is chosen from the group comprising phage coat proteins, preferably the proteins gene 3 protein (g3p) or gene 8 protein (g8p);
b) cloning said nucleotide sequence obtained at (a) in a phage-type vector, expression of said nucleotide sequence being dependent on a promoter of the phage, making it possible for said sequence obtained at (a) to be expressed in a bacterium and for particles of phage containing said nucleotide sequence obtained at (a) to be synthesized;
c) transfecting a bacterium with the vector obtained in step (b), said bacterium preferably being E. coli; and
d) isolating the particles of the phage from the culture supernatant.

3. A method according to claim 2, **characterized in that** the phage-type vector of step (b) is the phage fd-med.

4. A method of preparing a phage expressing one or more peptide-MHC complexes at its surface, said method being **characterized in that** it comprises the following steps:
a) preparing a nucleotide sequence comprising firstly a sequence coding successively for a signal peptide, an antigenic peptide, a linker peptide, and one or more extracellular domains of one or more products of the MHC, and, secondly a functional sequence which makes it possible to present the fusion protein as coded by said nucleotide sequence at the surface of a phage, and which is chosen from the group comprising phage coat proteins, preferably the proteins g3p or g8p;
b) cloning said nucleotide sequence obtained at (a) in a phagemid-type vector, expression of said nucleotide sequence being dependent on a promoter of the phagemid, making it possible for said sequence obtained at (a) to be expressed in a bacterium;
c) transfecting a bacterium with the vector obtained in step (b), said bacterium preferably being E. coli;
d) infecting said bacteria by means of a helper phage, making it possible to generate phages presenting the peptide-MHC complex(es) at their surfaces; and
e) isolating the particles of the phage from the culture supernatant.

5. A method according to claim 4, **characterized in that** the phagemid-type vector of step (b) is the phagemid pUC119.

6. A method according to claim 4 or claim 5, **characterized in that** the helper phage is the phage VCSM13.

7. A method according to any one of claims 2 to 6, **characterized in that** the step of isolating the particles of the phage from the culture supernatant is performed in the following steps:
(i) precipitating phage particles from the culture supernatant; and
(ii) putting the precipitate containing said particles of phage back in suspension in a suitable solution.

8. A method of preparing a phage expressing one or more peptide-MHC complexes at its surface according to any one of claims 2 to 7, **characterized in that** said method further comprises a step (g) of purifying the phages expressing the peptide-MHC complex(es) at their surfaces, preferably by immunoprecipitation using antibodies specifically directed against the peptide-MHC complex(es).

9. A method according to claim 4 or claim 8, **characterized in that** said phagemid is expressed under the dependence of an inducible promoter, preferably the inducible promoter of the Lac gene.

10. A method of preparing a phage expressing one or more peptide-MHC complexes according to any one of claims 2 to 9, said method being **characterized in that** said peptide-MHC complex(es) is/are associated non-covalently, and **in that**:
(i) the nucleotide sequence prepared during step (a) contains no sequence coding for the domains of the MHC, the sequence of the linker peptide being connected directly to the functional sequence;
(ii) the sequence coding for one or more products of the MHC is either (i) cloned in the vector in which the cloning of step (b) is performed, while also being dissociated from the nucleotide sequence prepared during step (a), or else (ii) cloned in another expression vector; and
(iii) the vector(s) is/are transfected in the same bacterium, as in step (c).

11. The use of a phage according to claim 1 for tagging and sorting specific T cells of one or more peptide-MHC complexes.

12. A method of tagging and sorting specific T cells of one or more peptide-MHC complexes, **characterized in that** it comprises the following steps:
i) putting the following in the presence of each other:
an excess of phages, each presenting, at its surface, a peptide-MHC complex as defined in claim 1; and
a pool of T cells, each presenting, at its surfaces, a set of receptors;
ii) selecting the T cells whose receptors are linked specifically to the phages carrying the peptide-MHC complexes by removing, by any suitable means, the excess of phages not linked specifically;
iii) tagging the phages linked specifically to the T cell receptors selected in (ii), the tagging being by means of antibodies specific to said phage; and
iv) recovering the T cells whose receptors are linked specifically to the phage tagged in step (iii), the recovery being performed by any suitable means.

13. A pharmaceutical composition including a phage according to claim 1.

14. A bank of phages as defined in claim 1.

15. The use of a phage bank according to claim 14 for screening antigenic peptides using a given T cell receptor (TCR) .

16. A method of screening antigenic peptides, **characterized in that** it comprises the following steps:
i) putting the following in the presence of each other: a phage bank according to claim 14, and a defined T cell receptor (TCR), in soluble form or at the surfaces of T cells;
ii) selecting the phages presenting the peptide-MHC complex linked specifically to the T cell receptor (TCR) by removing the phages that are not linked specifically; and
iii) identifying in the phages selected in step (ii) that nucleotide sequence which is coding for the antigenic peptide.

## Patentansprüche

1. Genetisch veränderter Phage, **dadurch gekennzeichnet, dass** er an seiner Oberfläche einen oder mehrere CMH-Peptid-Komplex(e) ausdrückt.

2. Herstellungsverfahren eines an seiner Oberfläche einen oder mehrere CMH-Komplex(e) ausdrückenden Phagen, **dadurch gekennzeichnet, dass** das genannte Verfahren die folgenden Stufen umfasst:
a) Die Herstellung einer Nukleotidsequenz mit einerseits einer nacheinander für ein Signalpeptide, ein Antigenpeptid, ein Verbindungspeptid, einen oder mehrere extrazelluläre(n) Bereich(e) eines Produkts oder mehrerer Produkte des CMH kodierenden Nukleotidsequenz und andererseits eine Funktionssequenz, die es erlaubt, das durch die genannte Nukleotidsequenz kodierte Fusionsprotein an der Oberfläche eines Phagen in Kontakt zu bringen, wobei die genannte Funktionssequenz aus der Gruppe bestehend aus Phagenmantelproteinen, bevorzugt die Proteine g3p oder g8p, ausgewählten Gruppe ausgewählt wird;
b) Das Klonen der in (a) erhaltenen genannten Nukleotidsequenz in einem Vektor vom Typ Phage, wobei der Ausdruck der genannten Nukleotidsequenz einem Promotor des Phagen unterstellt ist, der den Ausdruck der genannten, in (a) erhaltenen Sequenz in einer Bakterie und die Synthese von die genannte, in (a) erhaltene Nukleotidsequenz enthaltenden Phagenpartikeln erlaubt;
c) die Transfektion einer Bakterie mit einem in der Stufe (b) erhaltenen Vektor, wobei die genannte Bakterie bevorzugt E. coli ist;
d) die Isolation der Partikel des Phagen ausgehend von dem Kulturüberstand.

3. Verfahren gemäß Anspruch 2, **dadurch gekennzeichnet, dass** der Vektor vom Typ Phage der Stufe (b) der Phage fd-med ist.

4. Herstellungsverfahren eines ans einer Oberfläche einen oder mehrere CMH-Peptid-Komplex(e) ausdrückenden Phagen, **dadurch gekennzeichnet, dass** das genannte Verfahren die folgenden Stufen umfasst:
a) die Herstellung einer Nukleotidsequenz mit einerseits einer nacheinander für ein Signalpeptid, ein Antigenpeptid, ein Verbindungspeptid, einen oder mehrere extrazelluläre(n) Bereich(e) eines Produkts oder mehrerer Produkte des CMH kodierenden Sequenz und andererseits eine Funktionssequenz, die es erlaubt, das durch die genannte Nukleotidsequenz kodierende Fusionsprotein an der Oberfläche eines Phagen in Kontakt zu bringen, wobei die genannte Funktionssequenz aus der aus den Phagenmantelproteinen, bevorzug die Proteine g3p oder g8p enthaltenden Gruppe ausgewählt ist;
b) Das Klonen der genannten, in (a) erhaltenen Nukleotidsequenz in einem Vektor vom Typ Phagemid, wobei der Ausdruck der genannten Nukleotidsequenz einem Promotor des Phagemiden unterstellt ist, der den Ausdruck der genannten, in (a) erhaltenen Bakterie erlaubt;
c) Die Transfektion einer Bakterie mit dem in Stufe (b) erhaltenen Vektor, wobei die genannte Bakterie bevorzugt E. coli ist;
d) Die Infektion der genannten Bakterien mithilfe eines so genannten Helper-Phagen, der die Erzeugung von den / die CMH-Peptid-Komplex(e) an ihrer Oberfläche aufweisenden Phagen erlaubt;
e) Die Isolierung der Partikel des Phagen ausgehend von dem Kulturüberstand.

5. Verfahren gemäß Anspruch 4, **dadurch gekennzeichnet, dass** der Vektor vom Typ Phagemid der Stufe (b) der Phagemid pUC119 ist.

6. Verfahren gemäß Anspruch 4 oder 5, **dadurch gekennzeichnet, dass** der so genannte Helper-Phage der Phage VCSM13 ist.

7. Verfahren gemäß Anspruch 2 bis 6, **dadurch gekennzeichnet, dass** die Isolierstufe der Partikel des Phagen ausgehend von dem Kulturüberstand gemäß den folgenden Stufen erfolgt:
(i) dem Fällen der Phagenpartikel ausgehend von dem Kulturüberstand;
(ii) dem erneuten Ansetzen in Suspension der die genannten Phagenpartikel enthaltenden Fällung in einer geeigneten Lösung.

8. Herstellungsverfahren eines an seiner Oberfläche einen oder mehrere CMH-Peptid-Komplexe (s) gemäß Anspruch 2 bis 7 ausdrückenden Phagen, **dadurch gekennzeichnet, dass** das genannte Verfahren eine Reinigungsstufe (g) der an ihrer Oberfläche den oder die CMH-Peptid-Komplex(e) ausdrückenden Phagen umfasst, bevorzugt per Immunfällung mithilfe von speziell gegen den oder die CMH-Peptid-Komplex(e) gerichteten Antikörper.

9. Verfahren gemäß Anspruch 4 bis 8, **dadurch gekennzeichnet, dass** der Ausdruck des genannten Phagemiden in Abhängigkeit von einem induzierbaren Promotor erfolgt, bevorzugt des induzierbaren Promotors des Gens Lac.

10. Herstellungsverfahren eines an seiner Oberfläche einen oder mehrere CMH-Peptid-Komplex(e) gemäß Anspruch 2 bis 9 ausdrückenden Phagen, **dadurch gekennzeichnet, dass** der oder die genannte(n) CMH-Peptid-Komplex(e) nicht kovalent zugeordnet ist oder sind, und dadurch, dass:
(i) die in der Stufe (a) hergestellte Nukleotidsequenz keinerlei für die Bereiche des CMH kodierende Sequenz enthält, wobei die Sequenz des Verbindungspeptids direkt mit der Funktionssequenz verbunden ist;
(ii) die für eines oder mehrere Produkt(e) des CMH kodierende Sequenz entweder (i) in dem Vektor geklont ist, in dem das Klonen der Stufe (b) erfolgt, und dabei gleichzeitig von der in der Stufe (a) hergestellten Nukleotidsequenz dissoziiert ist, oder (ii) in einem anderen Ausdrucksvektor geklont ist;
(iii) der oder die Vektor(en) gemäß der Stufe (c) in ein und derselben Bakterie transfiziert ist oder sind.

11. Einsatz eines Phagen gemäß Anspruch 1 für die Kennzeichnung und das Sortieren von spezifischen T-Zellen eines oder mehrerer CMH-Peptid-Komplexes oder CMH-Peptid-Komplexe.

12. Kennzeichnungs- und Sortierverfahren von spezifischen T-Zellen eines CMH-Peptid-Komplexes oder mehrerer CMH-Peptid-Komplexe, **dadurch gekennzeichnet, dass** es die folgenden Stufen umfasst:
i) in Kontakt gebracht wird:
ein an seiner Oberfläche einen CMH-Peptid-Komplex aufweisenden Phagen gemäß Definition in Anspruch 1,
einen "Pool" von an ihrer Oberfläche eine Rezeptorenstruktur aufweisenden T-Zellen,
ii) Es werden die T-Zellen ausgewählt, deren Rezeptoren spezifisch mit den die CMH-Peptid-Komplexe tragenden Phagen verbunden sind, indem durch jedes geeignete Mittel der nicht speziell verbundene Phagenüberschuss ausgesondert wird,
iii) Es werden die speziell mit den in der Stufe (ii) ausgewählten T-Zellenrezeptoren verbundenen gekennzeichneten Phagen mithilfe des spezifischen Antikörpers des genannten Phagen gekennzeichnet,
iv) Es werden durch jedes geeignete Mittel die T-Zellen aufgefangen, deren Rezeptoren speziell mit dem in der Stufe (i-ii) gekennzeichneten Phagen verbunden sind.

13. Pharmazeutische Verbindung mit einem Phagen gemäß Anspruch 1.

14. Phagenbank gemäß Definition in Anspruch 1.

15. Einsatz einer Phagenbank gemäß Anspruch 14 für das Aussieben von Antigenpeptiden mithilfe eines bestimmten T (TCR)-Zellrezeptors.

16. Aussiebverfahren von Antigenpeptiden, **dadurch gekennzeichnet, dass** es die folgenden Stufen umfasst:
i) Es werden eine Phagenbank gemäß Anspruch 14 und ein definierter T (TCR)-Zellenrezeptor in löslicher Form oder an der Oberfläche von T-Zellen in Kontakt gebracht,
ii) Es werden den spezifisch mit dem T (TCR)-Zellenrezeptor verbundene CMH-Peptid-Komplex aufweisende Phagen durch Aussondern der Phagen ausgewählt, die nicht speziell verbunden sind.
iii) Es wird in den in der Stufe (a) ausgewählte Phagen die für das Antigenpeptid kodierende Nukleotidsequenz festgestellt.
